# EUROPEAN PATENT APPLICATION

(11) **EP 4 592 401 A2**
(43) Date of publication of application: **30.07.2025**
(21) Application number: 25183100.4
(22) Date of filing: 16.09.2021
(51) Int. Cl.: C12Q 1/6841

(54) **CHEMICAL COMPOSITIONS AND METHODS OF USING THE SAME**

(30) Priority: 16.09.2020 US 202063078965 P
(62) Divisional of application: 21787265.4
(71) Applicant: Bruker Spatial Biology, Inc., Billerica, MA 01821 (US)
(72) Inventor: The designation of the inventor has not yet been filed
(74) Representative: D Young & Co LLP

(57) **Abstract**

The present disclosure provides compositions and methods for the detection and identification of target nucleic acids within a tissue sample using fluorescent probes, wherein the probes comprise a target-binding domain and a barcode domain.

## Description

### CROSS-REFERENCE TO RELATED APPLICATIONS

This application claims priority to, and the benefit of, U.S. Provisional Application No. 63/078,965, filed on September 16, 2020. The contents of the aforementioned patent application are incorporated herein by reference in their entirety, for all purposes.

### BACKGROUND

Although there are currently a variety of methods for detecting nucleic acids and proteins in a biological sample, a need remains for improved, accurate, rapid, and sensitive multiplexed detection, identification, and quantification of target nucleic acids and proteins within a biological sample. Specifically, there is a need for the ability to detect the abundance and spatial location of specific nucleic acids and proteins within a tissue sample that has maintained its original morphology. The present disclosure addresses this need.

### SUMMARY

The present disclosure provides methods of determining the abundance and spatial position of at least two target analytes in a biological sample, wherein the biological sample is prepared by: i) contacting the biological sample with at least one nucleic acid probe by incubating the mounted biological sample with a solution comprising a plurality of ISH probes, wherein the solution comprises at least two species of ISH probes, wherein at least one species of ISH probe comprises a unique target binding domain that binds to one of at least two target analytes and a unique barcode domain specific for the target analyte, wherein the barcode domain comprises at least one attachment position; ii) washing the biological sample, the methods comprising: a) contacting the prepared biological sample with a plurality of reporter probes, wherein each reporter probe comprises at least one detectable label, thereby hybridizing a reporter probe to an attachment region of a barcode domain of at least one ISH probe hybridized to a target analyte in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment position in the barcode domains of ISH probes hybridized to a target analyte in the biological have been hybridized to a reporter probe comprising at least one detectable label, thereby determining the abundance and spatial position of the at least two target analytes in the biological sample based on the sequence in which the detectable labels were recorded.

In some aspects of the methods of the present disclosure, the at least two target analytes are target nucleic acid molecules, and wherein the target binding domain is a single-stranded polynucleotide comprising a nucleic acid sequence that is complementary to a target nucleic acid, wherein the target binding domain is about 35 to about 40 nucleotides in length, and wherein the target binding domain comprises D-DNA, and wherein the barcode domain is a single-stranded polynucleotide comprising at least one attachment region, wherein each attachment region comprises about one attachment sequence, wherein each of the attachment sequences is about 14 nucleotides in length, and wherein the sequences of each of the attachment sequences are different, and wherein the barcode domain comprises L-DNA.

In some aspects of the methods of the present disclosure, the at least two target analytes are target protein molecules, and wherein the target binding domain comprises a protein, preferably wherein the protein is an antibody, or antigen binding fragment, that specifically binds to a target protein molecule.

In some aspects of the methods of the present disclosure, the barcode domain comprises: i) at least two; ii) at least three; iii) at least four; or iv) at least five attachment regions.

In some aspects of the methods of the present disclosure, the solution comprises at least one negative ISH probe that is designed not to specifically bind to any target analyte in the biological sample, preferably wherein the ISH probe comprises at least one Evaluation of the External RNA Controls Consortium (ERCC) sequence, or a complement thereof. In some aspects of the methods of the present disclosure, the negative ISH probe is used to determine the level of background noise in the biological sample.

In some aspects of the methods of the present disclosure, the reporter probes comprise L-DNA

In some aspects of the methods of the present disclosure, the reporter probes comprise: a primary nucleic acid molecule comprising a first domain, a second domain and a photocleavable linker located between the first domain and the second domain, wherein the second domain of the primary nucleic acid molecule is hybridized to about six secondary nucleic acid molecules, wherein each secondary nucleic acid molecule comprises a first domain, a second domain and a photocleavable linker located between the first domain and the second domain, wherein the first domain of each of the secondary nucleic acid molecules is hybridized to the second domain of the primary nucleic acid molecule, wherein the second domain of each of the secondary nucleic acid molecules is hybridized to about five tertiary nucleic acid molecules, wherein each of the tertiary nucleic acid molecules comprise at least one detectable label, and wherein the primary nucleic acid molecule, the secondary nucleic acid molecules, and the tertiary nucleic acid molecules comprise L-DNA.

In some aspects of the methods of the present disclosure, the at least one detectable label is a fluorescent moiety.

In some aspects of the methods of the present disclosure, the method further comprises prior to step (a): pretreating the biological sample by: i) incubating the biological sample in a Sulfo-NHS Acetate Blocking solution for about 15 minutes; ii) washing the biological sample with Reporter Wash Buffer; iii) incubating the biological sample in autofluorescence suppressor buffer and/or illuminating the biological sample with blue and/or UV light, thereby quenching sample autofluorescence via photobleaching; and iv) washing the biological sample with Reporter Wash Buffer.

In some aspects of the methods of the present disclosure, step (a) comprises incubating the biological sample with a solution comprising the reporter probes at a concentration of 5 nM, 8.75x SSPE solution, 0.5% Tween-20 and, optionally 0.1% RNase inhibitor, in DEPC-treated water for at least about 15 minutes.

In some aspects of the methods of the present disclosure, step (b) comprises washing the biological sample with Reporter Wash Buffer.

In some aspects of the methods of the present disclosure, step (c) comprises: i) immersing the biological sample in Imaging Buffer; and ii) imaging the biological sample to record the identity and spatial position of the detectable labels of the hybridized reporter probes.

In some aspects of the methods of the present disclosure, step (d) comprises: i) performing at least one of or both of: illuminating the biological sample with UV light sufficient to cleave photocleavable linker moieties in the hybridized reporter probes; and washing the biological sample with Strip Wash Buffer; optionally, step (d) further comprises: iii) immersing the biological sample in Imaging Buffer; and iv) imaging the sample to ensure that there are no remaining detectable labels.

In some aspects of the methods of the present disclosure, the method further comprises performing morphology scanning of the biological sample to determine one or more regions of interest, preferably wherein performing morphology scanning comprises at least one of: i) staining the biological sample with a membrane specific-fluorescent staining solution and imaging the biological sample to identify the spatial location of cellular membranes within the sample; ii) staining the biological sample with a nuclear-specific fluorescent staining solution and imaging the biological sample to identify the spatial location of cellular nuclei in the sample; and iii) performing cell segmentation.

In some aspects of the methods of the present disclosure, the biological sample is further prepared prior to contacting the biological sample with at least one nucleic acid probe by: aa) mounting a biological sample onto a functionalized microscope slide thereby producing a mounted biological sample, wherein the biological sample is a formalin fixed paraffin embedded (FFPE) microtome section; bb) baking the mounted biological sample; cc) deparaffinizing the mounted biological sample; dd) performing a target retrieval reaction on the mounted biological sample; ee) permeabilizing the mounted biological sample; ff) applying at least one fiducial marker to the mounted biological sample; and gg) fixing the mounted biological sample

In some aspects of the methods of the present disclosure, the method further comprises after step (ii), assembling the mounted biological sample into a flow cell.

In some aspects of the methods of the present disclosure, the functionalized microscope slide is a positively charged microscope, preferably wherein the functionalized microscope slide is a (3-Aminopropyl)trimethoxysilane (APTMS)-functionalized microscope slide.

In some aspects of the methods of the present disclosure, the biological sample is an FFPE microtome section of a human tissue sample.

In some aspects of the methods of the present disclosure, step (bb) comprises baking the mounted biological sample at about 60°C for about 1 hour.

In some aspects of the methods of the present disclosure, step (cc) comprises: i) incubating the mounted biological sample in a first solution of xylene for about 5 minutes; ii) incubating the mounted biological sample in a second solution of xylene for about 5 minutes; iii) incubating the mounted biological sample in a first 100% ethanol solution for about 2 minutes; iv) incubating the mounted biological sample in the second 100% ethanol solution for about 2 minutes; and v) drying the mounted biological sample at about 60°C for about 5 minutes.

In some aspects of the methods of the present disclosure, step (dd) comprises: i) incubating the mounted biological sample in target retrieval solution at about 100°C; ii) incubating the mounted biological sample in DEPC-treated water for about 15 seconds; iii) incubating the mounted biological sample in a solution of 100% ethanol for about 3 minutes; and iv) drying the mounted biological sample.

In some aspects of the methods of the present disclosure, the mounted biological sample is incubated in the target retrieval solution for a time period as put forth in Table 1.

In some aspects of the methods of the present disclosure, the target retrieval solution comprises TRIS and EDTA solution and has a pH of about 9.

In some aspects of the methods of the present disclosure, step (ee) comprises: i) incubating the mounted biological sample at about 40°C in a proteinase solution, wherein the proteinase solution comprises protease K; ii) washing the biological sample with a first aliquot of DEPC-treated water; and iii) washing the biological sample with a second aliquot of DEPC-treated water.

In some aspects of the methods of the present disclosure, the mounted biological sample is incubated in the proteinase K solution for a time period as put forth in Table 2.

In some aspects of the methods of the present disclosure, step (ff) comprises: i) incubating the mounted biological sample in a solution comprising at least one fiducial marker for about 5 minutes at about room temperature, wherein the solution comprising at least one fiducial marker is a solution comprising carboxylated microspheres stained in red, yellow, blue and/or green at a concentration of about 0.0005% to about 0.003% in 2x SSCT solution; and ii) washing the mounted biological with 1x PBS.

In some aspects of the methods of the present disclosure, step (gg) comprises i) incubating the mounted biological sample in a 10% NBF for about 1 minutes; ii) incubating the mounted biological sample in a first tris glycine buffered solution for about 5 minutes; iii) incubating the mounted biological sample in a second tris glycine buffered solution for about 5 minutes; and iv) incubating the mounted biological sample in 1x PBS for about 5 minutes.

In some aspects of the methods of the present disclosure, the method further comprises after step (gg), incubating the mounted biological sample in a blocking solution, wherein incubating the mounted biological sample in a blocking solution comprises: i) incubating the mounted biological sample in a Sulfo-NHS-acetate/Tween20 solution for about 15 minutes, wherein the Sulfo-NHS-acetate/Tween20 solution comprises about 100 mM Sulfo-NHS-acetate, about 0.5% Tween20 in about 100 mM sodium phosphate pH 8; and ii) incubating the mounted biological sample in 1x PBS for about 5 minutes.

In some aspects of the methods of the present disclosure, incubating the mounted biological sample with a solution comprising a plurality of ISH probes comprises: incubating the mounted biological sample with a solution comprising a plurality of ISH probes for about 16 to about 18 hours at about 37°C, thereby hybridizing at least one ISH probe to a target analyte in the biological sample.

In some aspects of the methods of the present disclosure, washing the biological sample comprises: i) incubating the mounted biological sample with a first 2x SSC solution; ii) incubating the mounted biological sample in a first formamide solution; iii) incubating the mounted biological sample with a second formamide solution; iv) incubating the mounted biological sample with a second 2x SSC solution; and v) incubating the mounted biological sample with a third 2x SSC solution.

Any of the above aspects or aspects described herein can be combined with any other aspect.

Unless otherwise defined, all technical and scientific terms used herein have the same meaning as commonly understood by one of ordinary skill in the art to which this disclosure belongs. In the Specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

Although methods and materials similar or equivalent to those described herein can be used in the practice or testing of the present disclosure, suitable methods and materials are described below. All publications, patent applications, patents, and other references mentioned herein are incorporated by reference in their entirety. The references cited herein are not admitted to be prior art to the claimed invention. In the case of conflict, the present Specification, including definitions, will control. In addition, the materials, methods, and examples are illustrative only and are not intended to be limiting. Other features and advantages of the disclosure will be apparent from the following detailed description and claim.

### BRIEF DESCRIPTION OF THE DRAWINGS

The above and further features will be more clearly appreciated from the following detailed description when taken in conjunction with the accompanying drawings.
FIG. 1 is a schematic diagram of an exemplary in situ hybridization (ISH) probe of the present disclosure.
FIG. 2 is a schematic diagram of an exemplary reporter probe of the present disclosure.
FIGs. 3A, 3B, 3C, 3D, 3E, 3F and 3H are exemplary schematics of the steps of a method of detecting the abundance and spatial location of more than one species of target nucleic acid in a biological sample
FIG. 4 shows a series of graphs comparing the abundance of RNA target analytes in various cells measured using the methods of the present disclosure and standard RNA-seq methods.
FIG. 5 shows images of individual target analytes detected in a biological sample comprising MDA-MB-468 cells using the methods of the present disclosure. FIG. 5 also shows the quantification of the number of transcripts per cell analyzed.
FIG. 6A shows images of individual target analytes detected in Melanoma FFPE tissue samples using the methods of the present disclosure.
FIG. 6B shows the results of cell typing analyses that can be performed using spatial abundance data collected using the methods of the present disclosure.
FIG. 6C shows the results cell interaction induced differential expression analyses that can be performed using spatial abundance data collected using the methods of the present disclosure.
FIG. 6D shows images of individual target analytes detected in Melanoma FFPE tissue samples using the methods of the present disclosure.
FIG. 6E shows images of individual target analytes detected in non-small cell lung cancer (NSCLC) FFPE tissue samples using the methods of the present disclosure.
FIG. 6F shows images of individual target analytes detected in renal cell carcinoma FFPE tissue samples using the methods of the present disclosure.
FIG. 6G shows images of individual target analytes detected in colorectal cancer (CRC) and tonsil FFPE tissue samples using the methods of the present disclosure.

### DETAILED DESCRIPTION

The present disclosure provides methods for preparing a biological sample for fluorescent imaging. The present disclosure also provides in situ hybridization (ISH) probes and reporter probes for use in the methods of the present disclosure, as well as kits comprising these ISH probes and reporter probes. The present disclosure also provides methods of determining the abundance and spatial position of at least two target nucleic acid molecules in a biological sample

### Methods of Sample Processing

In some aspects, the present disclosure provides a method of preparing a biological sample for fluorescent imaging, the method comprising: a) mounting a biological sample onto a functionalized microscope slide thereby producing a mounted biological sample, wherein the biological sample is a formalin fixed paraffin embedded (FFPE) microtome section; b) baking the mounted biological sample; c) deparaffinizing the mounted biological sample; d) performing a target retrieval reaction on the mounted biological sample; e) permeabilizing the mounted biological sample; f) applying at least one fiducial marker to the mounted biological sample; g) fixing the mounted biological sample; h) contacting the mounted biological sample with at least one nucleic acid probe; and i) washing the mounted biological sample.

In some aspects, the preceding methods can optionally further comprise j) dehydrating the mounted biological sample.

In some aspects, the preceding methods can further comprise, after step (i) or after step (j) assembling the mounted biological sample into a flow cell.

In some aspects, the preceding methods can further comprise after step (g) and before step (h), incubating the mounted biological sample in a blocking solution.

In some aspects, the preceding methods can further comprise, before or after any of the steps, illuminating the biological sample with blue and/or UV light, thereby quenching sample autofluorescence via photobleaching. In some aspects, any combination of UV and readout channel illumination can be used to quench sample autofluorescence via photobleaching. In some aspects, the illumination can be performed concurrently with any of the above steps, including, but not limited to step (h). In some aspects, the illumination can be performed using low-dose illumination over extended time periods.

In some aspects, a functionalized microscope slide can be a (3-Aminopropyl)trimethoxysilane (APTMS)-functionalized microscope slide. In some aspects, an APTMS functionalized microscope slide can prepared using the following method: a) cleaning a microscope slide using a plasma machine; b) incubating the microscope slide in a 0.5% APTMS solution for soaking for about 1 minute; c) sonicating the microscope slide in the 0.5% APTMS solution for about 10 seconds; d) repeating steps (b) and (c) twice such that the microscope slide is immersed in the 0.5% APTMS solution for about 3.5 minutes; e) rising the microscope slide with water at least 3 times; and f) drying the microscope slide under nitrogen.

In some aspects, a functionalized microscope slide can be any positively-charged microscope slide. As would be appreciated by the skilled artisan, non-limiting examples of commercially-available, positively-charged microscope slides include, but are not limited to poly-L-Lysin coated glass slide, Leica BOND Plus slides and Fisherbrand^{™} SuperFrost^{™} Plus slides.

In some aspects of the methods of the present disclosure, mounting a biological sample onto a functionalized microscope slide can comprise mounting the biological sample onto the functionalized microscope slide and drying the mounted biological sample for at least about 12 hours, or at least about 13 hours, or at least about 14 hours, or at least about 15 hours, or at least about 16 hours, or at least about 17 hours, or at least about 18 hours at room temperature.

In some aspects of the methods of the present disclosure, baking a mounted biological sample can comprise baking the mounted biological sample at least about 50°C, or at least about 55°C, or at least about 60°C, or at least about 65°C, or at least about 70°C, or at least about 75°C., or at least about 80°C. In some aspects, baking a mounted biological sample can comprise baking the mounted sample at about 60°C.

In some aspects of the methods of the present disclosure, baking a mounted biological sample can comprising baking the mounted biological sample for at least about 0.5 hours, or at least about 1 hour, or at least about 1.5 hours, or at least about 2 hours. In some aspects, baking a mounted biological sample can comprise baking the mounted biological sample for about 1 hour.

In some aspects of the methods of the present disclosure, baking a mounted biological sample can comprise baking the mounted biological sample at about 60°C for about 1 hour.

In some aspects of the methods of the present disclosure, deparaffinizing a mounted biological sample can comprise: a) incubating the mounted biological sample in a first solution of xylene for about 5 minutes; b) incubating the mounted biological sample in a second solution of xylene for about 5 minutes; c) incubating the mounted biological sample in a first 100% ethanol solution for about 2 minutes; d) incubating the mounted biological sample in the second 100% ethanol solution for about 2 minutes, and e) drying the mounted biological sample at about 60°C for about 5 minutes. In some aspects, the incubation in the first solution of xylene and/or the second solution of xylene can comprise agitating the mounted biological sample in the xylene solution, for example, by moving the biological sample up and down in the solution.

Without wishing to be bound by theory, since FFPE samples contain DNA molecules that are crosslinked to each other as well as to RNA and protein molecules, breakage of these crosslinks can facilitate the release of DNA for subsequent purification. Breakage of these crosslinks can be achieved by performing a target retrieval reaction on a biological sample, such as an FFPE sample. In a target retrieval reaction, the biological sample, such as the FFPE sample, can be incubated with a target retrieval solution, wherein the target retrieval solution is suitable for removing crosslinking between DNA, RNA and protein within the biological sample, thereby allowing for the recovery of analyzable biomolecules

In some aspects, a target retrieval solution can have a pH of about 8.0 to about 10.0. In some aspects, a target retrieval solution can have a pH of about 8.5 to about 9.5. In some aspects, a target retrieval solution can have a pH of about 9.0. In some aspects, a target retrieval solution can comprise a buffering agent. In some aspects, the buffering agent can be TRIS.

In some aspects, a target retrieval solution can comprise a chelator. In some aspects, the chelator can be ethylenediaminetetraacetic acid (EDTA). In some aspects a target retrieval solution can comprise about 0.1 to about 2 mM EDTA. In some aspects, a target retrieval solution can comprise about 0.5 to about 1.5 mM EDTA. In some aspects, a target retrieval solution can comprise about 1.0 mM EDTA.

In some aspects, a target retrieval solution can be a TRIS and EDTA solution. In some aspects, a target retrieval solution can be a solution of about 10 mM TRIS and about 1 mM EDTA at pH 9.0.

In some aspects, a target retrieval solution can be RNAscope^{®} Target Retrieval Solution (ACD).

In some aspects of the methods of the present disclosure, performing a target retrieval reaction on a mounted biological sample can comprise incubating the mounted biological sample in a target retrieval solution at about 100°C. In some aspects, the mounted biological sample is incubated in target retrieval solution at about 100°C for an amount of time specific to the type of mounted biological sample. In a non-limiting example wherein the mounted biological sample is a human breast tumor sample, the mounted biological sample can be incubated in target retrieval solution at about 100°C for about 15 minutes. Incubation times for different sample types are shown in Table 1. In some aspects, performing a target retrieval reaction can further comprise, after incubating the mounted biological sample in target retrieval solution, incubating the mounted biological sample in water for at least about 15 seconds; incubating the mounted biological sample in a solution of 100% ethanol for at least about 3 minutes; and drying the mounted biological sample. In some aspects, the water can be diethyl pyrocarbonate (DEPC)-treated water.

Accordingly, performing a target retrieval reaction on a mounted biological sample can comprise: a) incubating the mounted biological sample in target retrieval solution at about 100°C for a time period as put forth in Table 1; b) incubating the mounted biological sample in DEPC-treated water for about 15 seconds; c) incubating the mounted biological sample in a solution of 100% ethanol for about 3 minutes; and d) drying the mounted biological sample.

**Table 1. Incubation times in 1x target retrieval solution for various biological sample types**

| Species of Biological Sample | Tissue Type | Pathology | Incubation Time (minutes) |
|---|---|---|---|
| Mouse | Intestine | Normal | 15 |
| | Intestine | Tumor | 15 |
| | Embryo | Normal | 15 |
| | Brain | Normal | 15 |
| | Spleen | Normal | 15 |
| | Eye/Retina | Normal | 15 |
| | Liver | Normal | 30 |
| | Kidney | Normal | 15 |
| Human | Breast | Tumor | 15 |
| | Colon | Tumor | 15 |
| | Colon | Normal | 15 |
| | Lung | Tumor | 15 |
| | Lung | Normal | 15 |
| | Prostate | Tumor | 15 |
| | Prostate | Normal | 15 |
| | Lymph node | Tumor 15 | |
| | Lymph node | Normal | 15 |
| | Tonsil | Normal | 15 |
| | Pancreas | Normal | 15 |
| | Cervical | Cancer | 15 |
| | Cervical | Normal | 15 |
| | Cervical dysplasia | Abnormal | 15 |
| | Brain | Tumor | 15 |
| | Brain | Normal | 15 |
| | Head | Cancer | 15 |
| | Neck | Cancer | 15 |
| | Liver | Cancer | 15 |
| | Kidney | Normal | 15 |
| | Skin | Normal | 15 |
| | Melanoma | Tumor | 15 |
| | Nevus | Benign | 15 |
| | Placenta | Normal | 15 |
| | Skin (tissue microarray [TMA]) | Normal | 15 |
| | Breast TMA | Normal | 15 |
| | Melanoma TMA | Normal | 15 |
| | Nevus TMA | Benign | 15 |
| | Stomach TMA | Normal | 15 |
| | Stomach TMA | Tumor | 15 |
| | Cell pellets, fixed with 10% NBF | - | 15 |
| | HeLa cells, fixed with 10% Formaldehyde/PBS/ACD Control | - | 15 |
| | Cell Pellets (general) | - | 8 |

In some aspects of the methods of the present disclosure, permeabilizing the mounted biological sample can comprise incubating the mounted biological sample in a proteinase K solution.

In some aspects, the proteinase K solution can be a solution wherein the concentration of proteinase K is at least about 0.1 µg/mL, or at least about 0.25 µg/mL, or at least about 0.5 µg/mL, or at least about 0.75 µg/mL, or at least about 1 µg/mL, or at least about 1.25 µg/mL, or at least about 1.5 µg/mL, or at least about 1.75 µg/mL, or at least about 2 µg/mL. or at least about 2.25 µg/mL, or at least about 2.5 µg/mL, or at least about 2.75 µg/mL, or at least about 3 µg/mL, or at least about 3.25 µg/mL, or at least about 3.5 µg/mL, or at least about 3.75 µg/mL, or at least about 4 µg/mL, or at least about 4.25 µg/mL, or at least about 4.5 µg/mL, or at least about 4.75 µg/mL, or at least about 5 µg/mL. In some aspects, the proteinase K solution is a solution wherein the concentration of proteinase K is about 1 µg/mL. In some aspects, the proteinase K solution is a solution wherein the proteinase K is diluted into Phosphate Buffered Saline (PBS). In some aspects, the proteinase K solution is a solution wherein the proteinase K is diluted into protease cocktail, including, but not limited to ACD Protease Plus.

In some aspects, the PBS can comprise a combination of NaCL, KCl, Na₂HPO₄ and KH₂PO₄. In some aspects, the PBS can comprise a solution of 137 mM NaCl, 2.7 mM KCl, 8 mM Na₂HPO₄, and 2 mM KH₂PO₄ at pH 7.4. Accordingly, in some aspects a proteinase K solution can be a solution wherein the concentration of proteinase K is about 1 µg/mL in PBS, wherein the PBS comprises 137 mM NaCl, 2.7 mM KCl, 8 mM Na₂HPO₄, and 2 mM KH₂PO₄ at pH 7.4.

In some aspects, permeabilizing the mounted biological sample can comprise incubating the mounted biological sample in a proteinase K solution at about 40°C. In some aspects, permeabilizing the mounted biological sample can comprise incubating the mounted biological sample in a proteinase K solution at about 40°C for an amount of time specific to the type of mounted biological sample. In a non-limiting example wherein the mounted biological sample is a human breast tumor sample, the mounted biological sample can be incubated in a proteinase K solution at about 40°C for about 30 minutes. Incubation times for different sample types are shown in Table 2.

In some aspects, permeabilizing the mounted biological sample can comprise incubating the mounted biological sample at about 40°C in a proteinase solution In some aspects, permeabilizing the mounted biological sample can comprise incubating the mounted biological sample at about 40°C in a proteinase solution for an amount of time specific to the type of mounted biological sample. In a non-limiting example wherein the mounted biological sample is a human breast tumor sample, the mounted biological sample can be incubated at about 40°C in a proteinase solution for about 30 minutes. Incubation times for different sample types are shown in Table 2.

In some aspects, a proteinase solution can comprise a solution of protease K at a concentration of about 0.1 to about 5.0 ug/mL, or 0.1 to 5.0 ug/mL. In some aspects, a proteinase solution can comprise a solution of protease K at a concentration of about 0.1 to about 5.0 ug/mL, or 0.1 to 5.0 ug/mL in PBS. In some aspects, a proteinase solution can comprise a solution of protease K at a concentration of about 0.1 to about 5.0 ug/mL, or 0.1 to 5.0 ug/mL in a protease cocktail (*e.g.* ACD protease plus solution). In some aspects, a proteinase solution can comprise a protease cocktail known in the art, *e.g.* ACD protease plus solution.

**Table 2. Incubation times in proteinase solution for various biological sample types**

| Species of Biological Sample | Tissue Type | Pathology | Incubation Time (minutes) |
|---|---|---|---|
| Mouse | Intestine | Normal | 30 |
| | Intestine | Tumor | 30 |
| | Embryo | Normal | 30 |
| | Brain | Normal | 30 |
| | Spleen | Normal | 15 |
| | Eye/Retina | Normal | 30 |
| | Liver | Normal | 30 |
| | Kidney | Normal | 30 |
| Human | Breast | Tumor | 30 |
| | Colon | Tumor | 30 |
| | Colon | Normal | 30 |
| | Lung | Tumor | 30 |
| | Lung | Normal | 30 |
| | Prostate | Tumor | 30 |
| | Prostate | Normal | 30 |
| | Lymph node | Tumor | 30 |
| | Lymph node | Normal | 30 |
| | Tonsil | Normal | 30 |
| | Pancreas | Normal | 30 |
| | Cervical | Cancer | 30 |
| | Cervical | Normal | 30 |
| | Cervical dysplasia | Abnormal | 30 |
| | Brain | Tumor | 30 |
| | Brain | Normal | 30 |
| | Head | Cancer | 30 |
| | Neck | Cancer | 30 |
| | Liver | Cancer | 30 |
| | Kidney | Normal | 30 |
| | Skin | Normal | 30 |
| | Melanoma | Tumor | 30 |
| | Nevus | Benign | 30 |
| | Placenta | Normal | 30 |
| | Skin (tissue microarray [TMA]) | Normal | 30 |
| | Breast TMA | Normal | 30 |
| | Melanoma TMA | Normal | 30 |
| | Nevus TMA | Benign | 30 |
| | Stomach TMA | Normal | 30 |
| | Stomach TMA | Tumor | 30 |
| | Cell pellets, fixed with 10% NBF | - | 15 |
| | HeLa cells, fixed with 10% Formaldehyde/PBS/ACD Control | - | 15 |
| | Cell Pellets (general) | - | 15 |

In some aspects, incubating a mounted biological sample in a proteinase K solution can further comprise drawing a hydrophobic barrier around the mounted biological sample, for example, with a PAP pen.

In some aspects, permeabilizing a mounted biological sample can comprise incubating the mounted biological sample in a proteinase K solution in a container that has been lined with paper (*e.g.* kimwipes or a suitable alternative) that have been wet with DEPC-treated water and preheated to about 40°C for at least about 30 minutes.

In some aspects, permeabilizing a mounted biological sample can further comprise, after incubating the mounted biological sample in a proteinase K solution, washing the mounted biological sample with water. The water can be DEPC-treated water. In some aspects, washing the mounted biological sample with water can comprise washing the mounted biological sample with a first aliquot of DEPC-treated water and then washing the mounted biological sample with a second aliquot of DEPC-treated water.

Accordingly, permeabilizing a mounted biological sample can comprise: a) incubating the mounted biological sample in a proteinase K solution at about 40°C for a time period as put forth in Table 2, wherein the concentration of proteinase K in the proteinase K solution is about 1 µg/mL; b) washing the biological sample with a first aliquot of DEPC-treated water; and c) washing the biological sample with a second aliquot of DEPC-treated water.

Accordingly, permeabilizing a mounted biological sample can comprise: a) incubating the mounted biological sample at about 40°C in a proteinase solution for a time period as put forth in Table 2, wherein the proteinase solution comprises a solution of protease K at a concentration of about 0.1 to about 5.0 ug/mL, or 0.1 to 5.0 ug/mL; b) washing the biological sample with a first aliquot of DEPC-treated water; and c) washing the biological sample with a second aliquot of DEPC-treated water.

In some aspects of the methods of the present disclosure, applying at least one fiducial marker to a mounted biological sample can comprise incubating the mounted biological sample in a solution comprising at least one fiducial marker. An at least one fiducial marker can be any fiducial marker known in the art to be useful for fluorescent imaging, as would be appreciated by the skilled artisan. In some aspects, the at least one fiducial marker can be diluted in 2x saline-sodium citrate (SSC) solution. In some aspects, the at least one fiducial marker can be diluted in 2x saline-sodium citrate tween (SSCT) solution. In some aspects, the mounted biological sample can incubated in the solution comprising at least one fiducial marker for at least about 1 minute, or at least about 2 minutes, or at least about 3 minutes, or at least about 4 minutes, or at least about 5 minutes, or at least about 6 minutes, or at least about 7 minutes, or at least about 8 minutes, or at least about 9 minutes, or at least about 10 minutes. In some aspects, the mounted biological sample can be incubated in the solution comprising at least one fiducial marker for about 5 minutes. In some aspects, the mounted biological sample can be incubated in the solution comprising the at least one fiducial marker at about room temperature. In some aspects, after incubation with the solution comprising at least one fiducial marker, the mounted biological sample can be washed, for example, with phosphate buffered solution (PBS). In some aspects, prior to applying the solution comprising at least one fiducial marker to the mounted biological sample, the solution can be agitated (*e.g.* vortexed) for at least 30 seconds.

In some aspects of the methods of the present disclosure, 2x SSC buffer can comprise about 300 mM NaCl and about 30 mM sodium citrate. In some aspects of the methods of the present disclosure, 2x SSC buffer can comprise 300 mM NaCl and 30 mM sodium citrate.

In some aspects of the methods of the present disclosure, 2x SSCT buffer can comprise about 0.1% Tween20, about 300 mM NaCl and about 30 mM sodium citrate. In some aspects of the methods of the present disclosure, 2x SSCT buffer can comprise 0.1% Tween20, 300 mM NaCl and 30 mM sodium citrate.

In some aspects, the at least one fiducial marker can be a 200 nm carboxylated microsphere in red, blue, yellow and/or green. In some aspects, a solution comprising at least one fiducial marker can comprise 200 nm carboxylated microspheres in red, blue and/or green at a concentration of at least about 0.00025%, or at least about 0.0005%, or at about 0.00075%, or at least about 0.001%, or at least about 0.00125%, or at least about 0.0015%, or at least about 0.00175%, or at least about 0.002%, or at least about 0.005%, or at least about 0.01%. In some aspects, a solution comprising at least one fiducial marker can comprise 200 nm carboxylated microspheres in red, blue and/or green at a concentration of about 0.001%. In some aspects, the at least one fiducial marker can be a carboxylated microsphere (*e.g.* 200 nm carboxylated microspheres) stained in red, yellow, blue and/or green. In some aspects, a solution comprising at least one fiducial marker can comprise carboxylated microspheres stained in red, yellow, blue and/or green at a concentration of at least about 0.00025%, or at least about 0.0005%, or at about 0.00075%, or at least about 0.001%, or at least about 0.00125%, or at least about 0.0015%, or at least about 0.00175%, or at least about 0.002%, or at least about 0.005%, or at least about 0.01%. In some aspects, a solution comprising at least one fiducial marker can comprise carboxylated microspheres stained in red, yellow, blue and/or green at a concentration of about 0.001%. In some aspects, a solution comprising at least one fiducial marker can comprise carboxylated microspheres stained in red, yellow, blue and/or green at a concentration of about 0.0005% to about 0.003%, or 0.0005% to 0.003%.

In some aspects, the at least one fiducial marker can be a fluorescent nano-diamond (FND). In some aspects, and FND can be a non-carboxylated FND. In some aspects, a solution comprising at least one fiducial marker can comprise FNDs at a concentration of at least about 0.0001%, or at least about 0.00015%, or at least about 0.0002%, or at least about 0.00025%, or at least about 0.0003%, or at least about 0.00035%, or at least about 0.0004%, or at least about 0.00045%, or at least about 0.0005%, or at least about 0.00055%, or at least about 0.001%. In some aspects, a solution comprising at least one fiducial marker can comprise FNDs at a concentration of about 0.00045%.

In some aspects, a solution comprising at least one fiducial marker can comprise a combination of at least two fiducial markers. In a non-limiting example, a solution comprising at least one fiducial marker can comprise 200 nm carboxylated microspheres in red, blue and/or green and non-carboxylated FNDs. In a non-limiting example, a solution comprising at least one fiducial marker can comprise 200 nm carboxylated microspheres in red, blue and/or green at a concentration of about 0.001% and non-carboxylated FNDs at a concentration of about 0.00045%.

In some aspects, a solution comprising at least one fiducial marker can comprise a combination of at least two fiducial markers. In a non-limiting example, a solution comprising at least one fiducial marker can comprise carboxylated microspheres stained in red, yellow, blue and/or green and non-carboxylated FNDs. In a non-limiting example, a solution comprising at least one fiducial marker can comprise nm carboxylated microspheres stained in red, blue and/or green at a concentration of about 0.0005% to about 0.003%, or 0.0005% to 0.003%, and non-carboxylated FNDs at a concentration of about 0.00045%.

In some aspects, a solution comprising at least on fiducial marker can be prepared by diluting the at least one fiducial marker in a suitable buffer solution, including, but not limited to 2x SSC solution, and then agitating (*e.g.* vortexing) the solution for about 1 minute, then sonicating the solution for about 2 minutes, then agitating the solution again for about 1 minute, then sonicating the solution again for about 2 minutes.

In some aspects, a solution comprising at least on fiducial marker can be prepared by diluting the at least one fiducial marker in a suitable buffer solution, including, but not limited to 2x SSCT solution, and then agitating (*e.g.* vortexing) the solution for about 1 minute, then sonicating the solution for about 2 minutes, then agitating the solution again for about 1 minute, then sonicating the solution again for about 2 minutes.

Accordingly, applying at least one fiducial marker to a mounted biological sample can comprise: a) incubating the mounted biological sample in a solution comprising at least one fiducial marker for about 5 minutes at about room temperature, wherein the solution comprising at least one fiducial marker is a solution comprising carboxylated microspheres in red, blue and/or green at a concentration of about 0.001% and non-carboxylated FNDs at a concentration of about 0.00045% in 2x SSC solution; and b) washing the mounted biological with 1x PBS.

Accordingly, applying at least one fiducial marker to a mounted biological sample can comprise: a) incubating the mounted biological sample in a solution comprising at least one fiducial marker for about 5 minutes at about room temperature, wherein the solution comprising at least one fiducial marker is a solution comprising carboxylated microspheres stained in red, yellow, blue and/or green at a concentration of about 0.0005% to about 0.003%, or 0.0005% to 0.003%; and b) washing the mounted biological with 1x PBS.

In some aspects of the methods of the present disclosure, fixing a mounted biological sample can comprise incubating the mounted biological sample in neutral buffered formalin (NBF) solution, then incubating the mounted biological sample in a tris glycine buffered solution, and then incubating the mounted biological sample in 1x PBS. In some aspects, the concentration of NBF in the NBF solution can be at least about 5%, or at least about 10%, or at least about 15%, or at least about 20%. In some aspects, the concentration of NBF in the NBF solution can be about 10%. In some aspects, any of the incubation steps in the fixing of the mounted biological sample can be for at least about 1 minute, or at least about 2 minutes, or at least about 3 minutes, or at least about 4 minutes, or at least about 5 minutes, or at least about 6 minutes, or at least about 7 minutes, or at least about 8 minutes, or at least about 9 minutes, or at least about 10 minutes. In some aspects, any of the incubation steps in the fixing of the mounted biological sample can be about 1 minute, or about 2 minutes, or about 3 minutes, or about 4 minutes, or about 5 minutes, or about 6 minutes, or about 7 minutes, or about 8 minutes, or about 9 minutes, or about 10 minutes. In some aspects, any of the incubation steps can be for about 5 minutes. In some aspects, any of the incubation steps can be for about 1 minute. In some aspects, incubating the mounted biological sample in a tris glycine buffered solution can comprise incubating the mounted biological sample in a first tris glycine buffered solution followed by incubating the mounted biological sample in a second tris glycine buffered solution.

Accordingly, fixing a mounted biological sample can comprise: a) incubating the mounted biological sample in a 10% NBF for about 5 minutes; b) incubating the mounted biological sample in a first tris glycine buffered solution for about 5 minutes; c) incubating the mounted biological sample in a second tris glycine buffered solution for about 5 minutes; and d) incubating the mounted biological sample in 1x PBS for about 5 minutes.

Accordingly, fixing a mounted biological sample can comprise: a) incubating the mounted biological sample in a 10% NBF for about 1 minute; b) incubating the mounted biological sample in a first tris glycine buffered solution for about 5 minutes; c) incubating the mounted biological sample in a second tris glycine buffered solution for about 5 minutes; and d) incubating the mounted biological sample in 1x PBS for about 5 minutes.

In some aspects of the methods of the present disclosure, incubating the mounted biological sample in a blocking solution can comprise incubating the mounted biological sample in a Sulfo-NHS-acetate/Tween20 solution. In some aspects, a Sulfo-NHS-acetate/Tween20 solution can comprise about 100 mM Sulfo-NHS-acetate, about 0.5% Tween20 in about 100 mM sodium phosphate pH 8. In some aspects, a Sulfo-NHS-acetate/Tween20 solution can comprise 100 mM Sulfo-NHS-acetate, 0.5% Tween20 in 100 mM sodium phosphate pH 8. In some aspects, the mounted biological sample can be incubated in a Sulfo-NHS-acetate/Tween20 solution for at least about 5 minutes, or at least about 10 minutes, or at least about 15 minutes, or at least about 20 minutes. In some aspects, the mounted biological sample can be incubated in a Sulfo-NHS-acetate/Tween20 solution for about 5 minutes, or about 10 minutes, or about 15 minutes, or about 20 minutes. In some aspects, the mounted biological sample can be incubated in a Sulfo-NHS-acetate/Tween20 solution for about 15 minutes.

In some aspects of the methods of the present disclosure, incubating the mounted biological sample in a blocking solution can comprise, after incubating the mounted biological sample in a Sulfo-NHS-acetate/Tween20 solution, incubating the mounted biological sample in a 1x PBS for at least about 1 minute, or at least about 2 minutes, or at least about 3 minutes, or at least about 4 minutes, or at least about 5 minutes, or at least about 6 minutes, or at least about 7 minutes, or at least about 8 minutes, or at least about 9 minutes, or at least about 10 minutes. In some aspects of the methods of the present disclosure, incubating the mounted biological sample in a blocking solution can comprise, after incubating the mounted biological sample in a Sulfo-NHS-acetate Tween20 solution, incubating the mounted biological sample in a 1x PBS for about 1 minute, or about 2 minutes, or about 3 minutes, or about 4 minutes, or about 5 minutes, or about 6 minutes, or about 7 minutes, or about 8 minutes, or about 9 minutes, or about 10 minutes. In some aspects of the methods of the present disclosure, incubating the mounted biological sample in a blocking solution can comprise, after incubating the mounted biological sample in a Sulfo-NHS-acetate/Tween20 solution, incubating the mounted biological sample in a 1x PBS for about 5 minutes

Accordingly, incubating the mounted biological sample in a blocking solution can comprise: i) incubating the mounted biological sample in a Sulfo-NHS-acetate/Tween20 solution for about 15 minutes, wherein the Sulfo-NHS-acetate/Tween20 solution comprises about 100 mM Sulfo-NHS-acetate, about 0.5% Tween20 in about 100 mM sodium phosphate pH 8; and ii) incubating the mounted biological sample in 1x PBS for about 5 minutes.

In some aspects of the methods of the present disclosure contacting the mounted biological sample with at least one nucleic acid probe can comprise incubating the mounted biological sample with a solution comprising a plurality of ISH probes of the present disclosure. In some aspects, the mounted biological sample can be incubated with the solution comprising a plurality of ISH probes for at least about 12 hours, or at least about 13 hours, or at least about 14 hours, or at least about 15 hours, or at least about 16 hours, or at least about 17 hours, or least about 18 hours, or at least about 19 hours, or at least about 20 hours, or at least about 21 hours, or at least about 22 hours, or at least about 23 hours, or at least about 24 hours. In some aspects, the mounted biological sample can be incubated with the solution comprising a plurality of ISH probes for about 16 to about 18 hours.

In some aspects, the mounted biological sample can be incubated with the solution comprising a plurality of ISH probes at a temperature of at least about 35°C, or at least about 36°C, or at least about 37°C, or at least about 38°C, or at least about 39°C, or at least about 40°C. In some aspects, the mounted biological sample can be incubated with the solution comprising a plurality of ISH probes at a temperature of about 35°C.

In some aspects, the solution comprising a plurality of ISH probes of the present disclosure can comprise a single species of ISH probe. In some aspects, the solution comprising a plurality of ISH probes of the present disclosure can comprise at least about 2, or at least about 3, or at least about 4, or at least about 5, or at least about 6, or at least about 7, or at least about 8, or at least about 9, or at least about 10, or at least about 25, or at least about 50, or at least about 75, or at least about 100, or at least about 250, or at least about 500, or at least about 750, or at least about 1000, or at least about 5,000, or at least about 10,000, or at least about 15,000, or at least about 20,000, or at least about 50,000, or at least about 100,000, or at least about 500,000, or at least about 1,000,000 different species of ISH probes.

In some aspects, the concentration of at least one species of ISH probe in the plurality can be at least about 0.01 nM, or at least about 0.1 nM, or at least about 1 nM, or at least about 5 nM, or at least about 10 nM, or at least about 25 nM, or at least about 50 nM, or at least about 75 nM, or at least about 100 nM, or at least about 125 nM, or at least about 150 nM, or at least about 175 nM, or at least about 200 nM, or at least about 300 nM, or at least about 400 nM, or at least about 500 nM. In some aspects, the concentration of at least one species of ISH probe in the plurality can be about 0.01 nM, or about 0.1 nM, or about 1 nM, or about 5 nM, or about 10 nM, or about 25 nM, or about 50 nM, or about 75 nM, or about 100 nM, or about 125 nM, or about 150 nM, or about 175 nM, or about 200 nM, or about 300 nM, or about 400 nM, or about 500 nM. In some aspects, the concentration of at least one species of ISH probe in the plurality can be about 200 nM. In some aspects, the concentration of at least one species of ISH probe in the plurality can be about 1 nM.

In some aspects, the concentration of each species of ISH probe in the plurality can be at least about 0.01 nM, or at least about 0.1 nM, or at least about 1 nM, or at least about 5 nM, or at least about 10 nM, or at least about 25 nM, or at least about 50 nM, or at least about 75 nM, or at least about 100 nM, or at least about 125 nM, or at least about 150 nM, or at least about 175 nM, or at least about 200 nM, or at least about 300 nM, or at least about 400 nM, or at least about 500 nM. In some aspects, the concentration of each species of ISH probe in the plurality can be about 0.01 nM, or about 0.1 nM, or about 1 nM, or about 5 nM, or about 10 nM, or about 25 nM, or about 50 nM, or about 75 nM, or about 100 nM, or about 125 nM, or about 150 nM, or about 175 nM, or about 200 nM, or about 300 nM, or about 400 nM, or about 500 nM. In some aspects, the concentration of each species of ISH probe in the plurality can be about 200 nM. In some aspects, the concentration of each species of ISH probe in the plurality can be about 1 nM.

In some aspects, a solution comprising a plurality of ISH probes can comprise at least one species of ISH probe that comprise target binding domains that are designed not to specifically bind to any target analyte (*e.g.* target nucleic acid molecule and/or target protein molecule) in the biological sample. In some aspects, a solution comprising a plurality of ISH probes can comprise at least two species, or at least three species, or at least four species, or at least five species, or at least six species, or at least seven species, or at least eight species, or at least nine species, or at least ten species, or at least 50 species, or at least 100 species, or at least 1000 species of ISH probes that comprise target binding domains that are designed not to specifically bind to any target analyte (*e.g.* target nucleic acid molecule and/or target protein molecule) in the biological sample. These ISH probes that comprise target binding domains that are designed not to specifically bind to any target analyte are referred to herein as "negative ISH probes". A non-limiting example of a negative ISH probe is an ISH probe comprising a target binding domain that is a single-stranded nucleic acid, wherein the sequence of the single-stranded nucleic acid is designed such that it is not complementary to any known sequence specific to the biological sample being analyzed and/or complementary to any known sequence present on earth. As would be appreciated by the skilled artisan, examples of such sequences include those published by the Evaluation of the External RNA Controls Consortium (ERCC). Without wishing to be bound by theory, the use of these negative ISH probes in the methods of the present disclosure can allow the skilled artisan to determine the level of background noise in the results from a biological sample. As would be appreciated by the skilled artisan, since the negative ISH probes should not bind to any target analyte, any signal originating from a negative ISH probe that is recorded represents non-specific binding of ISH probes within the sample (*i.e.* background noise). In some aspects, the skilled artisan can use the level of background noise detected by negative ISH probes to more accurately determine the absolute abundance of target analytes within the biological sample.

In some aspects, the solution comprising a plurality of ISH probes can comprise the ISH probes diluted in buffer R.

In some aspects, buffer R can comprise at least one of dextran sulfate, bovine serum albumin (BSA), single-stranded DNA (ssDNA), saline-sodium citrate (SSC) and formamide. In some aspects, buffer R can comprise a combination of dextran sulfate, BSA, ssDNA, SSC and formamide. In some aspects, the single-stranded DNA can comprise salmon sperm DNA.

In some aspects, the ISH probes can be diluted in buffer R such that the final concentration of dextran sulfate is about 0.5% to about 4.5%, or about 1.5% to about 3.5%. In some aspects, the ISH probes can be diluted in buffer R such that the final concentration of dextran sulfate is about 2.5%.

In some aspects, the ISH probes can be diluted in buffer R such that the final concentration of BSA is about 0.01% to about 2%, or about 0.1% to about 1%. In some aspects, the ISH probes can be diluted in buffer R such that the final concentration of BSA is about 0.2%.

In some aspects, the ISH probes can be diluted in buffer R such that the final concentration of ssDNA is about 0.01 mg/ml to about 1 mg/ml, or about 0.05 mg/ml to about 0.5 mg/ml. In some aspects, the ISH probes can be diluted in buffer R such that the final concentration of ssDNA is about 0.1 mg/ml.

In some aspects, the ISH probes can be diluted in buffer R such that the final concentration of SSC is about 0.5x to about 3.5x or about 1x to about 3x. In some aspects, the ISH probes can be diluted in buffer R such that the final concentration of SSC is about 2x.

In some aspects, the ISH probes can be diluted in buffer R such that the final concentration of formamide is about 20% to about 60%, or about 30% to about 50%. In some aspects, the ISH probes can be diluted in buffer R such that the final concentration of formamide is about 40%

In some aspects, the ISH probes can be diluted in buffer R such that the final concentration of dextran sulfate is about 2.5%, the final concentration of BSA is about 0.2%, the final concentration of ssDNA is about 0.1 mg/ml, the final concentration of SSC is about 2x and the final concentration of formamide is about 40%.

In some aspects, the solution comprising a plurality of ISH probes can further comprise an RNase inhibitor, including, but not limited to, SUPERase-In^{™} RNAse inhibitor. The concentration of RNAse inhibitor can be about 0.1 Units/µl.

In some aspects, prior to incubating the mounted biological sample with the solution comprising a plurality of ISH probes, the ISH probes are first denatured by incubating the ISH probes at about 95°C for about 2 minutes and then immediately cooling the ISH probes on ice for about 1 minute.

In some aspects, the mounted biological sample can be incubated with the solution comprising a plurality of ISH probes in a container that has been rinsed with an RNAse inhibitor solution and that has been lined with paper (*e.g.* kimwipes or a suitable alternative) that have been wetted with DEPC-treated water.

Accordingly, contacting the mounted biological sample with at least one nucleic acid probe can comprise: a) incubating the mounted biological sample with a solution comprising a plurality of ISH probes of the present disclosure for about 16 to about 18 hours at about 37°C, wherein the solution comprises at least one species of ISH probe, wherein at least one species of ISH probe in the plurality is present at a concentration of about 200 nM.

In some aspects of the methods of the present disclosure, washing a mounted biological sample can comprise: a) incubating the mounted biological sample with first 2x SSC solution; b) incubating the mounted biological sample in a first formamide solution; c) incubating the mounted biological sample with a second formamide solution; d) incubating the mounted biological sample with a second 2x SSC solution; and e) incubating the mounted biological sample with a third 2x SSC solution.

In some aspects, a formamide solution in be a formamide in 2x SSC solution In some aspects, the concentration of formamide can be at least about 10%, or at least about 20%, or at least about 30%, or at least about 40%, or at least about 50%, or at least about 60%, or at least about 70% In some aspects the concentration of formamide can be about 50%. In some aspects, the mounted biological sample can be incubated with the first formamide solution and/or the second formamide solution for at least about 15 minutes, or at least about 20 minutes, or at least about 25 minutes, or at least about 30 minutes, or at least about 35 minutes, or at least about 40 minutes. In some aspects, the mounted biological sample can be incubated with the first formamide solution and/or the second formamide solution for about 25 minutes.

In some aspects, the mounted biological sample can be incubated with the second 2x SSC solution and/or the third 2x SSC solution for at least about 0.5 minutes, or at least about 1 minute, or at least about 1.5 minutes, or at least about 2.0 minutes, or at least about 2.5 minutes, or at least about 3.0 minutes, or at least about 3.5 minutes, or at least about 4.0 minutes, or at least about 4.5 minutes, or at least about 5 minutes. In some aspects, the mounted biological sample can be incubated with the second 2x SSC solution and/or the third 2x SSC solution for about 2 minutes.

Accordingly, washing a mounted biological sample can comprise: a) incubating the mounted biological sample with first 2x SSC solution; b) incubating the mounted biological sample in a first 50% formamide in 2x SSC solution for about 25 minutes; c) incubating the mounted biological sample with a second 50% formamide in 2x SSC solution for about 25 minutes; d) incubating the mounted biological sample with a second 2x SSC solution for about two minutes; and e) incubating the mounted biological sample with a third 2x SSC solution for about two minutes.

In some aspects of the methods of the present disclosure, dehydrating a mounted biological sample can comprise incubating the mounted biological sample in an ethanol gradient, as would be appreciated by the skilled artisan. In some aspects, incubating the mounted biological sample in an ethanol gradient can comprise: a) incubating the mounted biological sample in a 70% ethanol solution for about 3 minutes; b) incubating the mounted biological sample in a 85% ethanol solution for about 3 minutes; and c) incubating the mounted biological sample in a 100% ethanol solution for about 3 minutes.

In some aspects, a biological sample can be an FFPE microtome section that is at least about 1 µm, or at least about 2 µm, or at least about 3 µm, or at least about 4 µm, or at least about 5 µm, or at least about 6 µm, or at least about 7 µm, or at least about 8 µm, or at least about 9 µm, or at least about 10 µm thick. In some aspects, the biological sample is an FFPE microtome section that is about 5 µm thick.

In some aspects, the biological sample can be a tissue sample from any organ. In some aspects, the biological sample is a tissue sample from the Intestine, Embryo, Brain, Spleen, Eye, Retina, Liver, Kidney, Breast, Throat, Colon, Lung, Prostate, Lymph node, Tonsil, Pancreas, Cervix, Head, Neck, Liver, Skin, Nevus, Placenta or any other organ.

In some aspects, the biological sample can comprise non-cancerous cells. In some aspects, the biological sample can comprise cancerous cells. In some aspects, the biological sample can comprise a combination of both non-cancerous cells and cancerous cells. The cancerous cells can be from a carcinoma, lymphoma, blastoma, sarcoma, leukemia and germ cell tumors. The cancerous cells can be from a adrenocortical carcinoma, bladder urothelial carcinoma, breast invasive carcinoma, cervical squamous cell carcinoma, endocervical adenocarcinoma, cholangiocarcinoma, colon adenocarcinoma, lymphoid neoplasm diffuse large B-cell lymphoma, esophageal carcinoma, glioblastoma multiforme, head and neck squamous cell carcinoma, kidney chromophobe, kidney renal clear cell carcinoma, kidney renal papillary cell carcinoma, acute myeloid leukemia, brain lower grade glioma, liver hepatocellular carcinoma, lung adenocarcinoma, lung squamous cell carcinoma, mesothelioma, ovarian serous cystadenocarcinoma, pancreatic adenocarcinoma, pheochromocytoma, paraganglioma, prostate adenocarcinoma, rectum adenocarcinoma, sarcoma, skin cutaneous melanoma, stomach adenocarcinoma, testicular germ cell tumors, thyroid carcinoma, thymoma, uterine carcinosarcoma, uveal melanoma. Other examples include breast cancer, lung cancer, lymphoma, melanoma, liver cancer, colorectal cancer, ovarian cancer, bladder cancer, renal cancer or gastric cancer. Further examples of cancer include neuroendocrine cancer, non-small cell lung cancer (NSCLC), small cell lung cancer, thyroid cancer, endometrial cancer, biliary cancer, esophageal cancer, anal cancer, salivary, cancer, vulvar cancer, cervical cancer, Acute lymphoblastic leukemia (ALL), Acute myeloid leukemia (AML), Adrenal gland tumors, Anal cancer, Bile duct cancer, Bladder cancer, Bone cancer, Bowel cancer, Brain tumors, Breast cancer, Cancer of unknown primary (CUP), Cancer spread to bone, Cancer spread to brain, Cancer spread to liver, Cancer spread to lung, Carcinoid, Cervical cancer, Children's cancers, Chronic lyniphocytic leukemia (CLL), Chrome myeloid leukemia (CML), Colorectal cancer, Ear cancer, Endometrial cancer, Eye cancer, Follicular dendritic cell sarcoma, Gallbladder cancer, Gastric cancer, Gastro esophageal junction cancers, Germ cell tumors, Gestational trophoblastic disease (GIT)), Hairy cell leukemia, Head and neck cancer, Hodgkin lymphoma, Kaposi's sarcoma, Kidney cancer, Laryngeal cancer, Leukemia, Gastric linitis plastica. Liver cancer, Lung cancer, Lymphoma, Malignant schwannoma, Mediastinal germ cell tumors, Melanoma skin cancer, Men's cancer, Merkel cell skin cancer, Mesothelioma, Molar pregnancy, Mouth and oropharyngeal cancer. Myeloma, Nasal and paranasal sinus cancer, Nasopharyngeal cancer, Neuroblastoma. Neuroendocrine tumors, Non-Hodgkin lymphoma (NHL), Esophageal cancer, Ovarian cancer, Pancreatic cancer, Penile cancer, Persistent trophoblastic disease and choriocarcinoma, Pheochromocytoma, Prostate cancer, Pseudomyxoma peritonei, Rectal cancer. Retinoblastoma, Salivary gland cancer, Secondary' cancer, Signet cell cancer, Skin cancer, Small bowel cancer, Soft tissue sarcoma, Stomach cancer, T cell childhood non Hodgkin lymphoma (NHL), Testicular cancer, Thymus gland cancer. Thyroid cancer. Tongue cancer. Tonsil cancer. Tumors of the adrenal gland, Uterine cancer. Vaginal cancer, Vulval cancer, Wilms' tumor, Womb cancer and Gynaecological cancer. Examples of cancer also include, but are not limited to, Hematologic malignancies, Lymphoma, Cutaneous T-cell lymphoma. Peripheral T-cell lymphoma, Hodgkin's lymphoma, Non-Hodgkin's lymphoma, Multiple myeloma, Chrome lymphocytic leukemia, chronic myeloid leukemia, acute myeloid leukemia, Myelodysplastic syndromes, Myelofibrosis, Biliary tract cancer, Hepatocellular cancer, Colorectal cancer, Breast cancer, Lung cancer, Non-small cell lung cancer, Ovarian cancer, Thyroid Carcinoma, Renal Cell Carcinoma, Pancreatic cancer, Bladder cancer, skin cancer, malignant melanoma, merkel cell carcinoma, Uveal Melanoma or Glioblastoma multiforme.

The biological sample can be derived from any species, including, but not limited to, humans, mice, rats, dogs, cats, sheep, rabbits, cows, goats or any other species.

In situ hybridization (ISH) probes of the present disclosure

### Target binding domain

The present disclosure provides in situ hybridization (ISH) probes for use in the methods of the present disclosure.

An ISH probe can comprise a target binding domain and a barcode domain. In some aspects, the target binding domain is operably linked to the barcode domain.

In some aspects, a target binding domain can comprise a protein, a peptide, an aptamer, or a peptoid which specifically binds to a target analyte in a biological sample. In some aspects, the protein can be an antibody, or an antigen binding fragment thereof. In some aspects, the protein can be a lectin protein. In some aspects, the protein can be any carbohydrate-binding protein known in the art.

In some aspects, a target binding domain can be a single stranded polynucleotide. A target binding domain can comprise a sequence that is complementary to a target nucleic acid that is to be identified using the methods of the present disclosure.

In some aspects, a target binding domain in be at least about 35 nucleotides in length to at least about 40 nucleotides in length. In some aspects, a target binding domain can be about 35 nucleotides to about 40 nucleotides in length. In some aspects, a target binding domain can comprise about 20 nucleotides, or about 21 nucleotides, or about 22 nucleotides, or about 23 nucleotides, or about 24 nucleotides, or about 25 nucleotides, or about 26 nucleotides, or about 27 nucleotides, or about 28 nucleotides, or about 29 nucleotides, or about 30 nucleotides, or about 31 nucleotides, or about 32 nucleotides, or about 33 nucleotides, or about 34 nucleotides, or about 35 nucleotides, or about 36 nucleotides, or about 37 nucleotides, or about 38 nucleotides, or about 39 nucleotides, or about 40 nucleotides, or about 41 nucleotides, or about 42 nucleotides, or about 43 nucleotides, or about 45 nucleotides in length.

In some aspects, a target binding domain comprises D-DNA. In some aspects, a target binding domain consists of D-DNA.

In some aspects, a target binding domain can be about 35 nucleotides to about 40 nucleotides in length and comprises D-DNA. In some aspects, a target binding domain can be about 35 nucleotides to about 40 nucleotides in length and consists of D-DNA.

### Barcode domain

In some aspects, a barcode domain can be a single stranded polynucleotide.

A barcode domain can comprise at least one attachment region. In some aspects, a barcode domain can comprise at least two, at least three, at least four, at least five, at least six, at least seven, at least eight, at least nine, or at least ten attachment regions.

In some aspects, a barcode domain can comprise about 4 attachment regions.

An attachment region can comprise at least one nucleic acid sequence that is capable of being reversibly bound by a reporter probe of the present disclosure. A nucleic acid sequence that is capable of being reversibly bound by a reporter probe of the present disclosure is herein referred to as an attachment sequence. Accordingly, an attachment region of a barcode domain can comprise at least one attachment sequence. In some aspects, the attachment sequences within a single attachment region can be identical; thus, the reporter probes that bind within that single attachment region will be identical. In some aspects, the attachment sequences within a single attachment can be different; thus, the reporter probes that bind within that single attachment will be different.

In some aspects, wherein a barcode domain comprises more than one attachment region, the attachment sequences in each of the different attachment regions can be different; thus, different reporter probes will bind to each attachment region in the barcode domain.

In some aspects, an attachment sequence can be about 5 nucleotides, or about 6 nucleotides, or about 7 nucleotides, or about 8 nucleotides, or about 9 nucleotides, or about 10 nucleotides, or about 11 nucleotides, or about 12 nucleotides, or about 13 nucleotides, or about 14 nucleotides, or about 15 nucleotides, or about 16 nucleotides, or about 17 nucleotides, or about 18 nucleotides, or about 19 nucleotides, or about 20 nucleotides in length. In some aspects, an attachment sequence can be about 14 nucleotides in length.

In some aspects, a barcode domain comprises L-DNA. In some aspects, a barcode domain consists of L-DNA.

In some aspects, a barcode domain can comprise about 4 attachment regions, wherein each attachment region comprises about 1 attachment sequence, wherein each attachment sequence is about 14 nucleotides in length, such that the barcode domain is about 56 nucleotides in length, and wherein the nucleic acid sequence of each of the attachment sequences are different, wherein the barcode domain comprises L-DNA. In some aspects, a barcode domain can comprise about 4 attachment regions, wherein each attachment region comprises about 1 attachment sequence, wherein each attachment sequence is about 14 nucleotides in length, such that the barcode domain is about 56 nucleotides in length, and wherein the nucleic acid sequence of each of the attachment sequences are different, wherein the barcode domain consists of L-DNA.

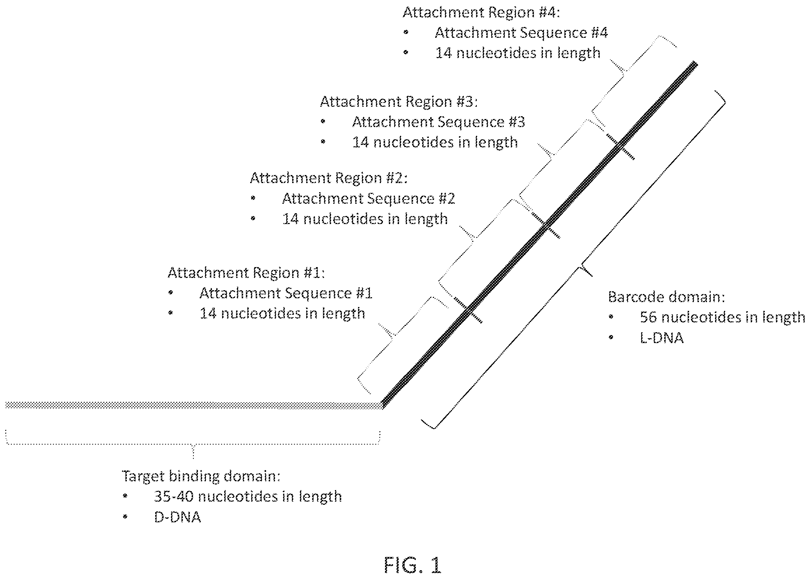

Accordingly, the present disclosure provides an ISH probe comprising a target binding domain and a barcode domain, wherein the target binding domain is a single-stranded polynucleotide comprising a nucleic acid sequence that is complementary to a target nucleic acid, wherein the target binding domain is about 35 to about 40 nucleotides in length, and wherein the target binding domain comprises D-DNA, and wherein the barcode domain is a single-stranded polynucleotide comprising about four attachment regions, wherein each attachment region comprises about one attachment sequence, wherein each of the attachment sequences is about 14 nucleotides in length, and wherein the sequences of each of the attachment sequences are different, and wherein the barcode domain comprises L-DNA. A schematic of this exemplary ISH probe is shown in FIG. 1.

Accordingly, the present disclosure provides an ISH probe comprising a target binding domain and a barcode domain, wherein the target binding domain is a single-stranded polynucleotide comprising a nucleic acid sequence that is complementary to a target nucleic acid, wherein the target binding domain is about 35 to about 40 nucleotides in length, and wherein the target binding domain consists of D-DNA, and wherein the barcode domain is a single-stranded polynucleotide comprising about four attachment regions, wherein each attachment region comprises about one attachment sequence, wherein each of the attachment sequences is about 14 nucleotides in length, and wherein the sequences of each of the attachment sequences are different, and wherein the barcode domain consists of L-DNA.

### Reporter probes of the present disclosure

The present disclosure provides reporter probes for use in the methods of the present disclosure. The reporter probes of the present disclosure bind to the attachment sequences within the attachment regions of the barcode domains of the ISH probes of the present disclosure. The reporter probes comprise at least one detectable label, *e.g.* a fluorescent moiety, that allows them to be detected in the methods of the present disclosure.

A reporter probe can comprise at least two domains, wherein the first domain hybridizes to an attachment sequence and the second domain comprises at least one detectable label.

In some aspects, a reporter probe can comprise at least about 10, or at least about 15, or at least about 20, or at least about 25, or at least about 30, or at least about 35, or at least about 40, or at least about 45, or at least about 50 detectable labels In some aspects, a reporter probe can comprise about 10, or about 15, or about 20, or about 25, or about 30, or about 35, or about 40, or about 45, or about 50 detectable labels.

In some aspects, a reporter probe can be pre-assembled prior to being contacted with a biological sample.

In some aspects, a reporter probe can comprise a primary nucleic acid molecule. A primary nucleic acid molecule can be a single-stranded polynucleotide. In some aspects, a primary nucleic acid molecule can comprise L-DNA. In some aspects, a primary nucleic acid molecule can consist of L-DNA.

A primary nucleic acid molecule can comprise at least two domains. In some aspects, the first domain of a primary nucleic acid molecule can hybridize to an attachment sequence in an attachment region of a barcode domain of an ISH probe of the present disclosure. In some aspects, the second domain of a primary nucleic acid molecule comprises at least one detectable label.

In some aspects, the second domain of a primary nucleic acid molecule can hybridize to at least one secondary nucleic acid molecule. In some aspects, a primary nucleic acid molecule can hybridize to at least about two, or at least about three, or at least about four, or at least about five, or at least about six, or at least about seven, or at least about eight, or at least about nine, or at least about ten secondary nucleic acid molecules. In some aspects, a primary nucleic acid molecule can hybridize to about 6 secondary nucleic acid molecules.

In some aspects, a primary nucleic acid molecule can further comprise a cleavable linker moiety. In some aspects, the cleavable linker moiety can be located between the first domain and the second domain, such that when the cleavable linker moiety is cleaved, the first domain and the second domain are separated. In preferred aspects, the cleavable linker moiety is a photocleavable linker moiety.

In some aspects, the first domain of a primary nucleic acid molecule can be about 5 nucleotides, or about 6 nucleotides, or about 7 nucleotides, or about 8 nucleotides, or about 9 nucleotides, or about 10 nucleotides, or about 11 nucleotides, or about 12 nucleotides, or about 13 nucleotides, or about 14 nucleotides, or about 15 nucleotides, or about 16 nucleotides, or about 17 nucleotides, or about 18 nucleotides, or about 19 nucleotides, or about 20 nucleotides in length. In some aspects, the first domain of a primary nucleic acid molecule can be about 14 nucleotides in length.

In some aspects, the second domain of a primary nucleic acid molecule can be about 75 nucleotides, or about 76 nucleotides, or about 77 nucleotides, or about 78 nucleotides, or about 79 nucleotides, or about 80 nucleotides, or about 81 nucleotides, or about 82 nucleotides, or about 83 nucleotides, or about 84 nucleotides, or about 85 nucleotides, or about 86 nucleotides, or about 87 nucleotides, or about 88 nucleotides, or about 89 nucleotides, or about 90 nucleotides in length. In some aspects, the second domain of a primary nucleic acid molecule can be about 84 nucleotides in length.

In some aspects, a primary nucleic acid molecule can be about 90 nucleotides, or about 91 nucleotides, or about 92 nucleotides, or about 93 nucleotides, or about 94 nucleotides, or about 95 nucleotides, or about 96 nucleotides, or about 97 nucleotides, or about 98 nucleotides, or about 99 nucleotides, or about 100 nucleotides, or about 101 nucleotides, or about 102 nucleotides, or about 103 nucleotides, or about 104 nucleotides, or about 105 nucleotides, or about 106 nucleotides, or about 107 nucleotides, or about 108 nucleotides, or about 109 nucleotides, or about 110 nucleotides in length. In some aspects, a primary nucleic acid can be about 98 nucleotides in length.

In some aspects, a reporter probe can comprise at least one secondary nucleic acid molecule. In some aspects, a reporter probe can comprise at least about two, or at least about three, or at least about four, or at least about five, or at least about six, or at least about seven, or at least about eight, or at least about nine, or at least about ten secondary nucleic acid molecules. In some aspects, a reporter probe can comprise about six secondary nucleic acid molecules. A secondary nucleic acid molecule can be a single-stranded polynucleotide. In some aspects, a secondary nucleic acid molecule can comprise L-DNA. In some aspects, a secondary nucleic acid molecule can consist of L-DNA.

A secondary nucleic acid molecule can comprise at least two domains. In some aspects, the first domain of a secondary nucleic acid molecule can hybridize to a primary nucleic acid molecule. In some aspects, the second domain of a secondary nucleic acid molecule can comprise at least one detectable label.

In some aspects, a secondary nucleic acid molecule can further comprise a cleavable linker moiety. In some aspects, the cleavable linker moiety can be located between the first domain and the second domain, such that when the cleavable linker moiety is cleaved, the first domain and the second domain of the secondary nucleic acid molecule are separated. In preferred aspects, the cleavable linker moiety is a photocleavable linker moiety.

In some aspects, the second domain of a secondary nucleic acid molecule can hybridize to at least one tertiary nucleic acid molecule. In some aspects, the second domain of a secondary nucleic acid molecule can hybridize to at least about two, or at least about three, or at least about four, or at least about five, or at least about six, or at least about seven, or at least about eight, or at least about nine, or at least about ten tertiary nucleic acid molecules. In some aspects, the second domain of a secondary nucleic acid molecule can hybridize to about five tertiary nucleic acid molecules.

In some aspects, the first domain of a secondary nucleic acid molecule can be about 5 nucleotides, or about 6 nucleotides, or about 7 nucleotides, or about 8 nucleotides, or about 9 nucleotides, or about 10 nucleotides, or about 11 nucleotides, or about 12 nucleotides, or about 13 nucleotides, or about 14 nucleotides, or about 15 nucleotides, or about 16 nucleotides, or about 17 nucleotides, or about 18 nucleotides, or about 19 nucleotides, or about 20 nucleotides in length. In some aspects, the first domain of a secondary nucleic acid molecule can be about 14 nucleotides in length.

In some aspects, the second domain of a secondary nucleic acid molecule can be about 65 nucleotides, or about 66 nucleotides, or about 67 nucleotides, or about 68 nucleotides, or about 69 nucleotides, or about 70 nucleotides, or about 71 nucleotides, or about 72 nucleotides, or about 73 nucleotides, or about 74 nucleotides, or about 75 nucleotides, or about 76 nucleotides, or about 77 nucleotides, or about 78 nucleotides, or about 79 nucleotides, or about 80 nucleotides, or about 81 nucleotides, or about 82 nucleotides, or about 83 nucleotides, or about 84 nucleotides, or about 85 nucleotides in length. In some aspects, the second domain of a secondary nucleic acid molecule can be about 75 nucleotides in length.

In some aspects, a reporter probe can comprise at least one tertiary nucleic acid molecule. In some aspects, a reporter probe can comprise at least about 20, or at least about 21, or at least about 22, or at least about 23, or at least about 24, or at least about 25, or at least about 26, or at least about 27, or at least about 28, or at least about 29, or at least about 30, or at least about 31, or at least about 32, or at least about 33, or at least about 34, or at least about 35, or at least about 36, or at least about 37, or at least about 38, or at least about 39, or at least about 40 tertiary nucleic acid molecules. In some aspects, a reporter probe can comprise about 30 tertiary nucleic acid molecules.

In some aspects, a tertiary nucleic acid molecule can comprise a domain that hybridizes to a secondary nucleic acid molecule.

In some aspects, a tertiary nucleic acid molecule can comprise at least one detectable label.

In some aspects, a tertiary nucleic acid molecule can be about 5 nucleotides, or about 6 nucleotides, or about 7 nucleotides, or about 8 nucleotides, or about 9 nucleotides, or about 10 nucleotides, or about 11 nucleotides, or about 12 nucleotides, or about 13 nucleotides, or about 14 nucleotides, or about 15 nucleotides, or about 16 nucleotides, or about 17 nucleotides, or about 18 nucleotides, or about 19 nucleotides, or about 20 nucleotides, or about 21 nucleotides, or about 22 nucleotides, or about 23 nucleotides, or about 24 nucleotides, or about 25 nucleotides in length. In some aspects, a tertiary nucleic acid molecule can be about 15 nucleotides in length.

In some aspects wherein a reporter probe comprises more than one detectable label, all of the detectable labels of the reporter probe can have the same emission spectrum. In aspects wherein the detectable labels are fluorescent labels, reporter probes wherein all of the detectable labels have the same emission spectrum can be referred to as "single-color" reporter probes.

In some aspects wherein a reporter probe comprises more than one detectable label, the reporter probe can have two or more detectable labels that each have a different emission spectra. In aspects wherein the detectable labels are fluorescent labels, reporter probes that have two or more detectable labels that each have a different emission spectra can be referred to as "multicolor" reporter probes.

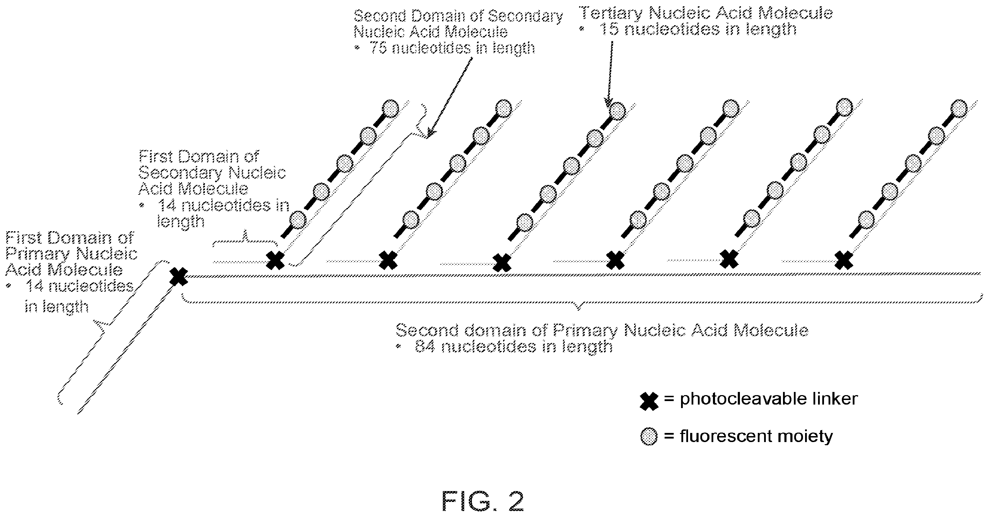

The present disclosure provides a reporter probe comprising a primary nucleic acid molecule comprising a first domain, a second domain and a photocleavable linker located between the first domain and the second domain, wherein the second domain of the primary nucleic acid molecule is hybridized to about six secondary nucleic acid molecules, wherein each secondary nucleic acid molecule comprises a first domain, a second domain and a photocleavable linker located between the first domain and the second domain, wherein the first domain of each of the secondary nucleic acid molecules is hybridized to the second domain of the primary nucleic acid molecule, wherein the second domain of each of the secondary nucleic acid molecules is hybridized to about five tertiary nucleic acid molecules, wherein each of the tertiary nucleic acid molecules comprise at least one detectable label, and wherein the primary nucleic acid molecule, the secondary nucleic acid molecules, and the tertiary nucleic acid molecules comprise L-DNA. A schematic of this exemplary reporter probe is shown in FIG. 2. In some aspects, the first domain of the primary nucleic acid molecule is about 14 nucleotides in length, the second domain of the primary nucleic acid molecule is about 84 nucleotides in length, the first domain of the secondary nucleic acid molecules is about 14 nucleotides in length, the second domain of the secondary nucleic acid molecules is about 75 nucleotides in length, and each of the tertiary nucleic acid molecules is about 15 nucleotides in length.

The present disclosure provides a reporter probe comprising a primary nucleic acid molecule comprising a first domain, a second domain and a photocleavable linker located between the first domain and the second domain, wherein the second domain of the primary nucleic acid molecule is hybridized to about six secondary nucleic acid molecules, wherein each secondary nucleic acid molecule comprises a first domain, a second domain and a photocleavable linker located between the first domain and the second domain, wherein the first domain of each of the secondary nucleic acid molecules is hybridized to the second domain of the primary nucleic acid molecule, wherein the second domain of each of the secondary nucleic acid molecules is hybridized to about five tertiary nucleic acid molecules, wherein each of the tertiary nucleic acid molecules comprise at least one detectable label, and wherein the primary nucleic acid molecule, the secondary nucleic acid molecules, and the tertiary nucleic acid molecules consists of L-DNA. In some aspects, the first domain of the primary nucleic acid molecule is about 14 nucleotides in length, the second domain of the primary nucleic acid molecule is about 84 nucleotides in length, the first domain of the secondary nucleic acid molecules is about 14 nucleotides in length, the second domain of the secondary nucleic acid molecules is about 75 nucleotides in length, and each of the tertiary nucleic acid molecules is about 15 nucleotides in length.

In some aspects, a photocleavable moiety can be cleaved upon exposure to UV light. The light can be provided by a light source selected from the group consisting of an arc-lamp, a laser, a focused UV light source, and light emitting diode.

A cleavable linker moiety can be 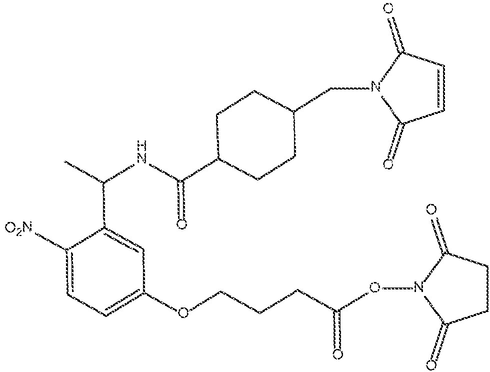 or a stereoisomer or salt thereof

A cleavable linker moiety can be 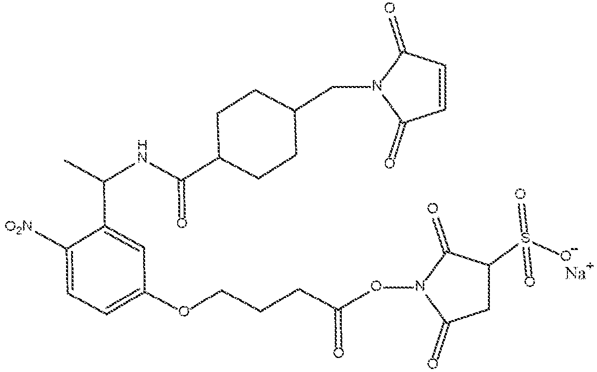 or a stereoisomer or salt thereof.

A cleavable linker moiety can be

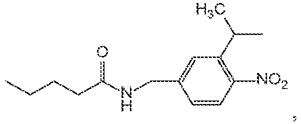

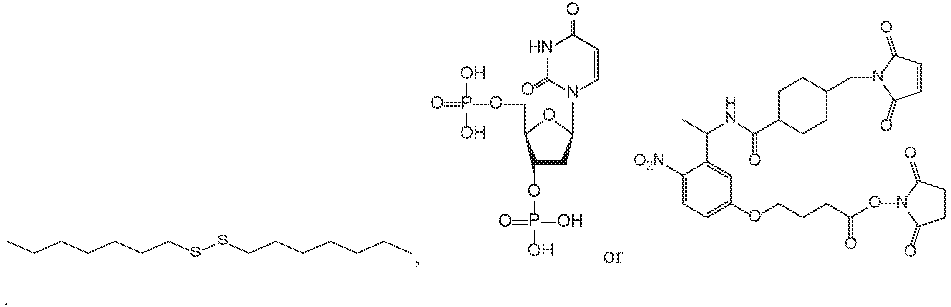

A cleavable linker moiety can be 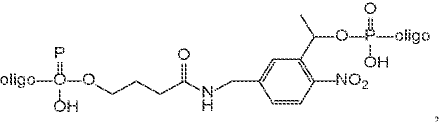 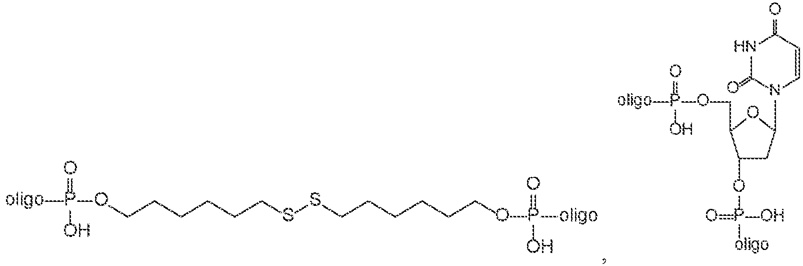 or

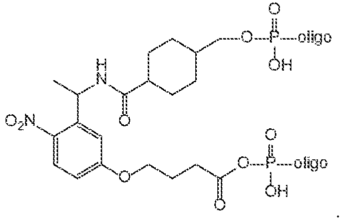

A cleavable linker moiety can be

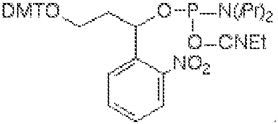

In preferred aspects, a detectable label can be a fluorescent moiety or a fluorescent label. One of skill in the art can consult references directed to labeling nucleic acids. Examples of fluorescent moieties include, but are not limited to, yellow fluorescent protein (YFP), green fluorescent protein (GFP), cyan fluorescent protein (CFP), red fluorescent protein (RFP), umbelliferone, fluorescein, fluorescein isothiocyanate, rhodamine, dichlorotriazinylamine fluorescein, cyanines, dansyl chloride, phycocyanin, phycoerythrin and the like.

Fluorescent labels and their attachment to nucleotides and/or oligonucleotides are described in many reviews, including Haugland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991). Particular methodologies applicable to the disclosure are disclosed in the following sample of references: U.S. Patent Nos. 4,757,141; 5,151,507; and 5,091,519. One or more fluorescent dyes can be used as labels for labeled target sequences, *e.g.*, as disclosed by U.S. Patent Nos. 5,188,934 (4,7-dichlorofluorescein dyes); 5,366,860 (spectrally resolvable rhodamine dyes); 5,847,162 (4,7-dichlororhodamine dyes); 4,318,846 (ether-substituted fluorescein dyes); 5,800,996 (energy transfer dyes); Lee et al. 5,066,580 (xanthine dyes); 5,688,648 (energy transfer dyes); and the like. Labelling can also be carried out with quantum dots, as disclosed in the following patents and patent publications: U.S. Patent Nos. 6,322,901; 6,576,291; 6,423,551; 6,251,303; 6,319,426; 6,426,513; 6,444,143; 5,990,479; 6,207,392; 2002/0045045; and 2003/0017264. As used herein, the term "fluorescent label" comprises a signaling moiety that conveys information through the fluorescent absorption and/or emission properties of one or more molecules. Such fluorescent properties include fluorescence intensity, fluorescence lifetime, emission spectrum characteristics, energy transfer, and the like.

Commercially available fluorescent nucleotide analogues readily incorporated into nucleotide and/or oligonucleotide sequences include, but are not limited to, Cy3-dCTP, Cy3-dUTP, Cy5-dCTP, Cy5-dUTP (Amersham Biosciences, Piscataway, NJ), fluorescein- 12-dUTP, tetramethylrhodamine-6-dUTP, TEXAS RED^{™}-5-dUTP, CASCADE BLUE^{™}-7-dUTP, BODIPY TMFL-14-dUTP, BODIPY TMR-14-dUTP, BODIPY TMTR-14-dUTP, RHODAMINE GREEN^{™}-5-dUTP, OREGON GREENR^{™} 488-5-dUTP, TEXAS RED^{™}- 12-dUTP, BODIPY TM 630/650- 14-dUTP, BODIPY TM 650/665- 14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546- 14-dUTP, fluorescein- 12-UTP, tetramethylrhodamine-6-UTP, TEXAS RED^{™}-5-UTP, mCherry, CASCADE BLUE^{™}-7-UTP, BODIPY TM FL-14-UTP, BODIPY TMR-14-UTP, BODIPY TM TR-14-UTP, RHODAMINE GREEN^{™}-5-UTP, ALEXA FLUOR^{™} 488-5-UTP, LEXA FLUOR^{™} 546- 14-UTP (Molecular Probes, Inc. Eugene, OR) and the like. Alternatively, the above fluorophores and those mentioned herein can be added during oligonucleotide synthesis using for example phosphoroamidite or NHS chemistry. Protocols are known in the art for custom synthesis of nucleotides having other fluorophores (*See,* Henegariu et al. (2000) Nature Biotechnol. 18:345). 2-Aminopurine is a fluorescent base that can be incorporated directly in the oligonucleotide sequence during its synthesis. Nucleic acid could also be stained, a priori, with an intercalating dye such as DAPI, YOYO- 1 , ethidium bromide, cyanine dyes (*e.g.,* SYBR Green) and the like.

Other fluorophores available for post-synthetic attachment include, but are not limited to, ALEXA FLUOR^{™} 350, ALEXA FLUOR^{™} 405, ALEXA FLUOR^{™} 430, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, Pacific Orange, rhodamine 6G, rhodamine green, rhodamine red, tetramethyl rhodamine, Texas Red (available from Molecular Probes, Inc., Eugene, OR), Cy2, Cy3, Cy3.5, Cy5, Cy5.5, Cy7 (Amersham Biosciences, Piscataway, NJ) and the like. FRET tandem fluorophores can also be used, including, but not limited to, PerCP-Cy5.5, PE-Cy5, PE-Cy5.5, PE-Cy7, PE-Texas Red, APC-Cy7, PE-Alexa dyes (610, 647, and 680), APC-Alexa dyes and the like.

Metallic silver or gold particles can be used to enhance signal from fluorescently labeled nucleotide and/or oligonucleotide sequences (Lakowicz et al. (2003) BioTechniques 34:62).

Other suitable labels for an oligonucleotide sequence can include fluorescein (FAM, FITC), digoxigenin, dinitrophenol (DNP), dansyl, biotin, bromodeoxyuridine (BrdU), hexahistidine (6xHis), phosphor-amino acids (*e.g.,* P-tyr, P-ser, P-thr) and the like. The following hapten/antibody pairs can be used for detection, in which each of the antibodies is derivatized with a detectable label: biotin/a-biotin, digoxigenin/a-digoxigenin, dinitrophenol (DNP)/a-DNP, 5-Carboxyfluorescein (FAM)/a-FAM.

Detectable labels described herein are spectrally resolvable. "Spectrally resolvable" in reference to a plurality of fluorescent labels means that the fluorescent emission bands of the labels are sufficiently distinct, *i.e.,* sufficiently non-overlapping, that molecular tags to which the respective labels are attached can be distinguished on the basis of the fluorescent signal generated by the respective labels by standard photodetection systems, *e.g.,* employing a system of band pass filters and photomultiplier tubes, or the like, as exemplified by the systems described in U.S. Patent Nos. 4,230,558; 4,811,218; or the like, or in Wheeless et al., pgs. 21-76, in Flow Cytometry: Instrumentation and Data Analysis (Academic Press, New York, 1985). Spectrally resolvable organic dyes, such as fluorescein, rhodamine, and the like, means that wavelength emission maxima are spaced at least 20 nm apart, and in another aspect, at least 40 nm apart. For chelated lanthanide compounds, quantum dots, and the like, spectrally resolvable means that wavelength emission maxima are spaced at least 10 nm apart, or at least 15 nm apart.

### Imaging methods

The present disclosure provides a method of detecting the abundance and spatial location of more than one species of target analyte in a biological sample using the ISH probes and reporter probes of the present disclosure.

In some aspects, a target analyte can be a nucleic acid (*i.e.* a target nucleic acid or a target nucleic acid molecule). Accordingly, the present disclosure provides a method of detecting the abundance and spatial location of more than one species of target nucleic acid in a biological sample using the ISH probes and reporter probes of the present disclosure.

In some aspects, a target analyte can be a protein (*i.e.* a target protein or target protein molecule). Accordingly, the present disclosure provides a method of detecting the abundance and spatial location of more than one species of target protein in a biological sample using the ISH probes and reporter probes of the present disclosure.

In some aspects, a target analyte can be a carbohydrate molecule (*e.g.* a sugar moiety, specific glycosylation motifs, etc.). Accordingly, the present disclosure provides a method of detecting the abundance and spatial location of more than one species of target carbohydrate molecule.

A target nucleic acid can be any nucleic acid to which an ISH probe of the present disclosure can hybridize. The target nucleic acid can be DNA or RNA. In preferred aspects, a target nucleic acid is an mRNA.

In brief, each species of target nucleic acid that is to be detected in a biological sample is assigned a predetermined and unique ISH probe that comprises: a) a target binding domain that is complementary to that specific species of target nucleic acid (*i.e.* that is designed such that it only hybridizes to that specific species of target nucleic acid); and b) a unique barcode domain comprising a unique nucleic acid sequence that is specific to that species of target nucleic acid. The unique nucleic acid sequence of the barcode domain is designed such that a specific sequence of reporter probes of the present disclosure will bind sequentially to the different attachment regions in the barcode domain, thereby creating a "linear order of detectable labels" which is specific to that species of target nucleic acid.

A target protein can be any protein to which an ISH probe of the present disclosure can bind.

In brief, each species of target protein that is to be detected in a biological sample is assigned a predetermined and unique ISH probe that comprises: a) a target binding domain that binds to that specific species of target protein (*i.e.* that is designed such that it only binds to that specific species of protein); and b) a unique barcode domain comprising a unique nucleic acid sequence that is specific to that species of protein. The unique nucleic acid sequence of the barcode domain is designed such that a specific sequence of reporter probes of the present disclosure will bind sequentially to the different attachment regions in the barcode domain, thereby creating a "linear order of detectable labels" which is specific to that species of target protein.

A target carbohydrate molecule can be any carbohydrate molecule to which an ISH probe of the present disclosure can bind. In some aspects, the target carbohydrate molecule can be part of a specific glycosylation motif In some aspects, the target carbohydrate can be part of a specific lipid to be detected.

In brief, each species of target carbohydrate that is to be detected in a biological sample is assigned a predetermined and unique ISH probe that comprises: a) a target binding domain that binds to that specific species of target carbohydrate (*i.e.* that is designed such that it only binds to that specific species of carbohydrate); and b) a unique barcode domain comprising a unique nucleic acid sequence that is specific to that species of carbohydrate. The unique nucleic acid sequence of the barcode domain is designed such that a specific sequence of reporter probes of the present disclosure will bind sequentially to the different attachment regions in the barcode domain, thereby creating a "linear order of detectable labels" which is specific to that species of target carbohydrate.

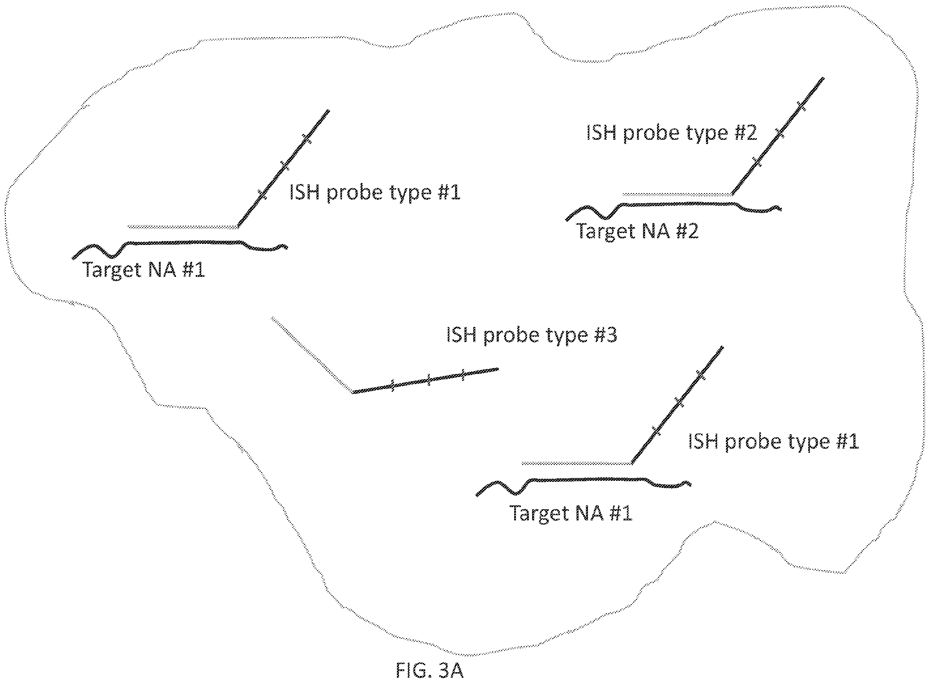

A schematic of a non-limiting example of these methods is shown in FIGs. 3A-3H, which shows the detection of two different species of target nucleic acids in a biological sample using the ISH probes of the present disclosure and reporter probes of the present disclosure. The method begins in FIG. 3A with a biological sample that comprises two copies of target nucleic acid #1 (one in the upper left part of the sample and one in the lower right part of the sample) and one copy of target nucleic acid #2 (in the upper right part of the biological sample. In the first step of the method, the biological sample is contacted with a plurality of ISH probes of the present disclosure. The ISH probes with target binding domains that are complementary to target nucleic acid #1 (ISH probe type #1) hybridize to target nucleic acid #1 and ISH probes with target binding domains that are complementary to target nucleic acid #2 (ISH probe type #2) hybridize to target nucleic acid #2. A third type of probe (ISH probe type #3), which has a target binding domain complementary to a third type of target nucleic acid does not hybridize within the biological sample, because the biological sample does not contain the third type of target nucleic acid.

In the second step, the non-hybridized ISH probes are washed off of the biological sample.

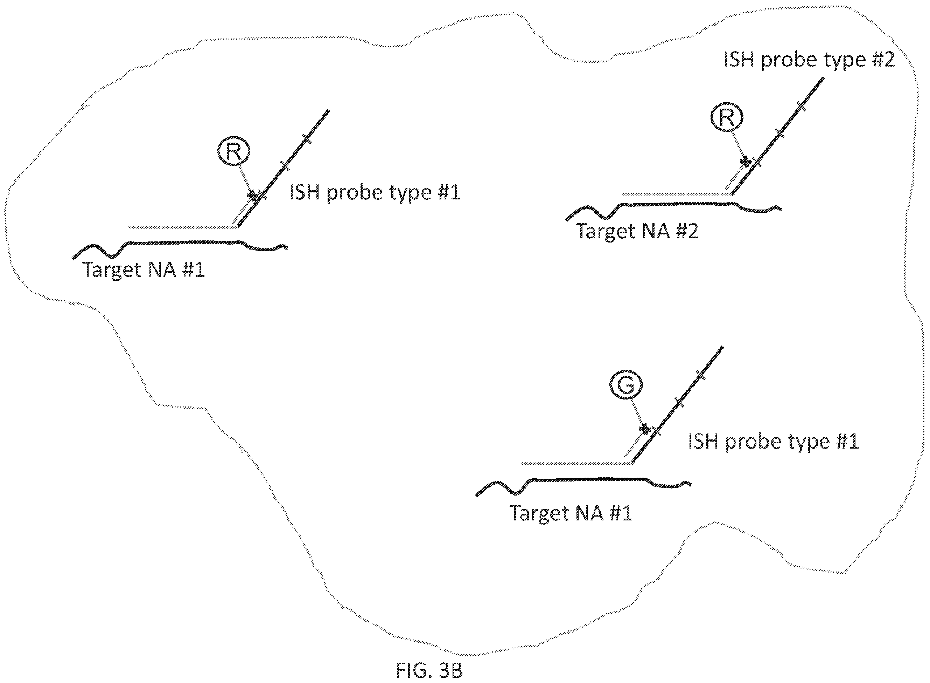

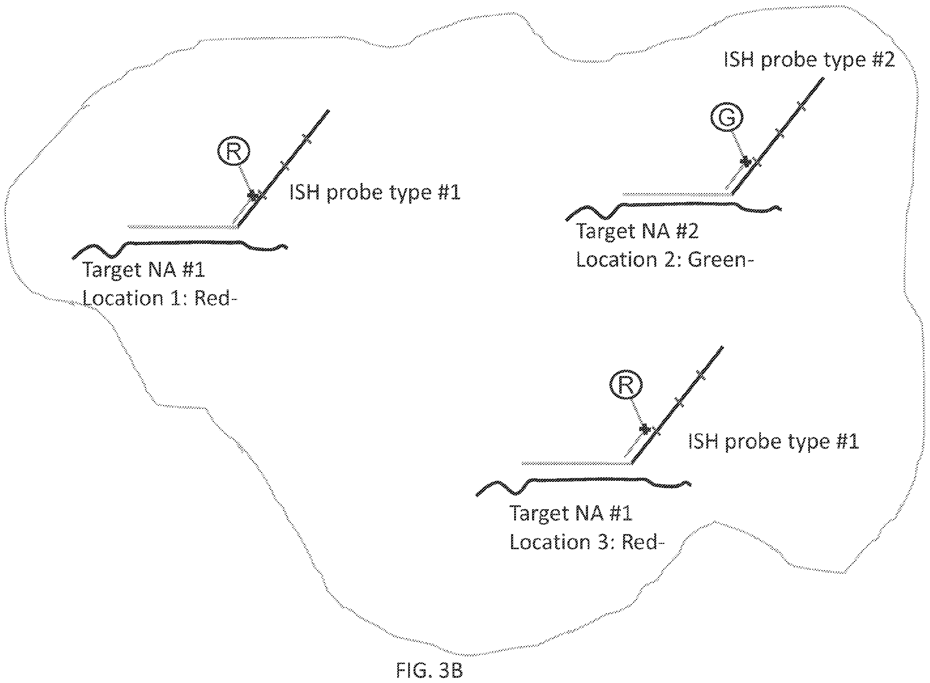

In a third step, shown in FIG. 3B, the biological sample is contacted with a plurality of reporter probes comprising detectable labels. In this non-limiting example, the detectable labels are fluorescent labels. The barcode domain of ISH probe type #1 is designed such that the first attachment region hybridizes to a reporter probe with a red fluorescent label and ISH probe type #2 is designed such that the first attachment region hybridizes to a reporter probe with a green fluorescent label

In a fourth step, shown in FIG. 3C, the identity and spatial location of the detectable labels of the hybridized reporter probes are recorded. Accordingly, during the first round of imaging, a red label was detected in "Location 1", a green label was detected in "Location 2" and a red label was detected in "Location 3".

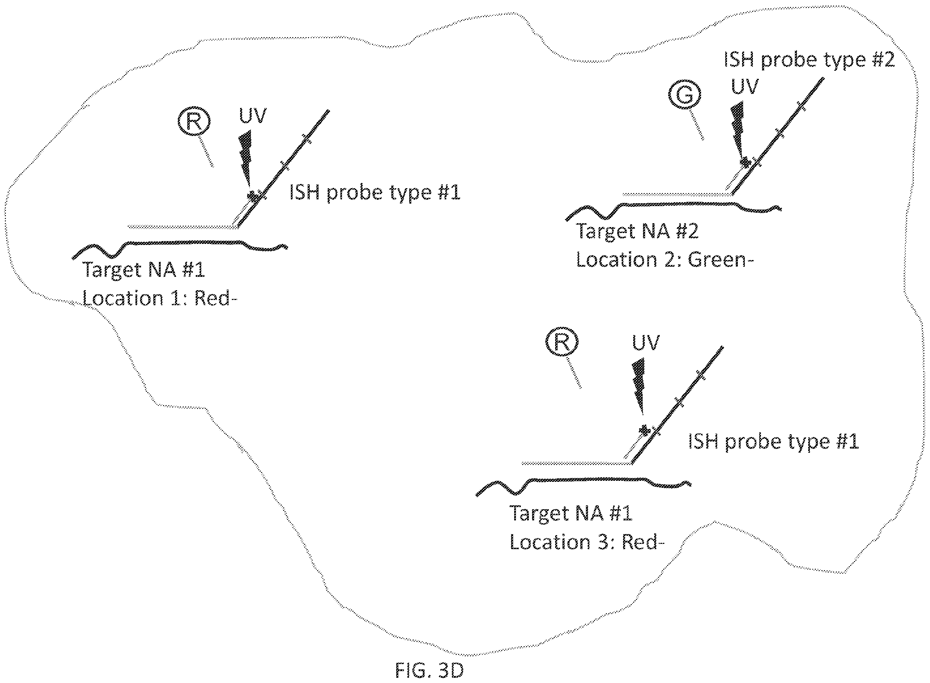

In a fifth step, shown in FIG. 3D, the detectable labels are removed from the hybridized reporter probes. In this non-limiting example, the reporter probes comprise photocleavable moieties that can be cleaved by illumination with UV light, which releases the detectable labels, which are subsequently washed away.

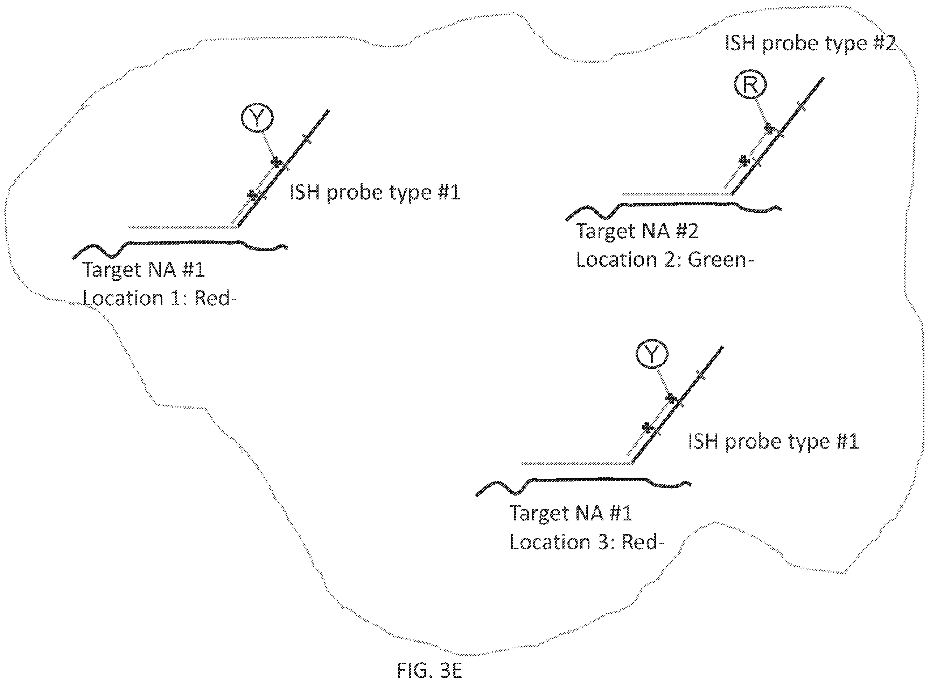

In a sixth step, shown in FIG. 3E, the biological sample is contacted with a second plurality of reporter probes comprising detectable labels. The barcode domain of ISH probe type #1 is designed such that the second attachment region hybridizes to a reporter probe with a yellow fluorescent label and ISH probe type #2 is designed such that the second attachment region hybridizes to a reporter probe with a red fluorescent label.

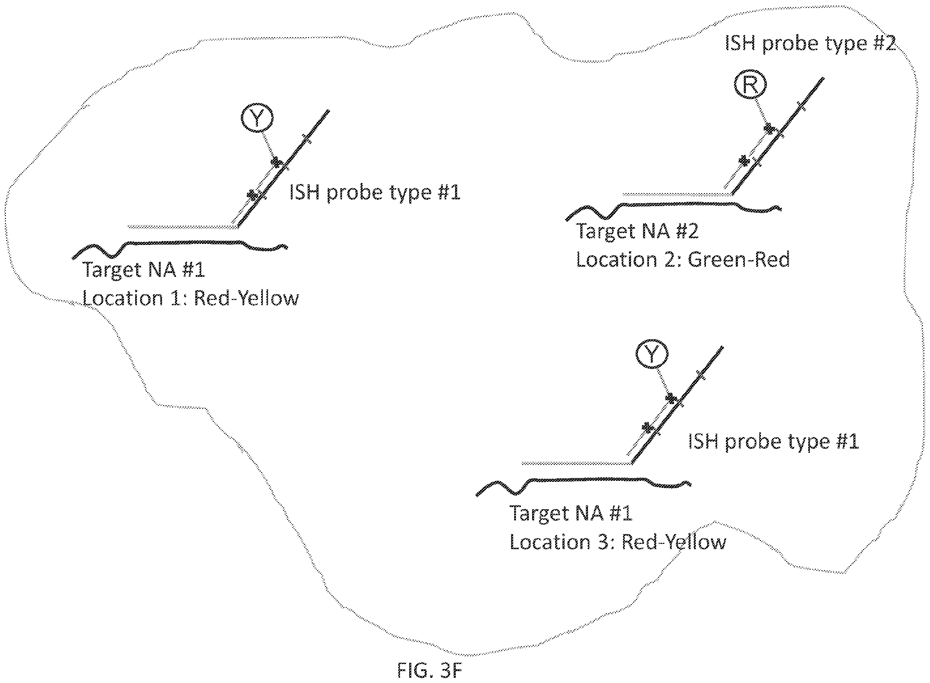

In a seventh step, as shown in FIG. 3F, the identity and spatial location of the detectable labels of the hybridized reporter probes are recorded. Accordingly, during the second round of imaging, a yellow label was detected in Location 1, a red label was detected in Location 2 and a yellow label was detected in Location 3.

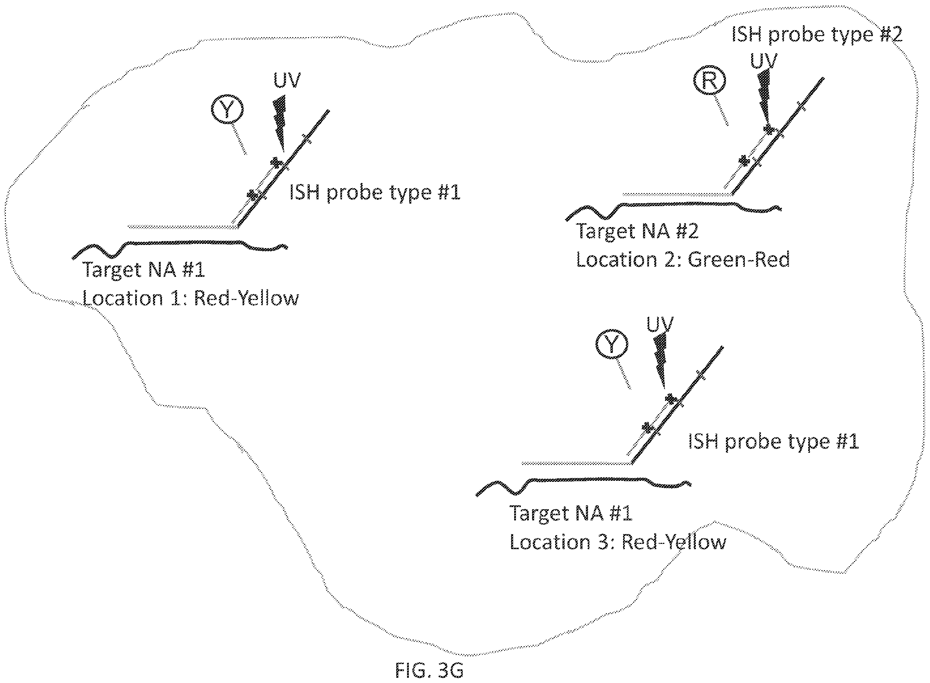

In an eighth step, as shown in FIG. 3G the detectable labels are removed from the hybridized reporter probes by UV-induced cleavage of photocleavable moieties within the reporter probes.

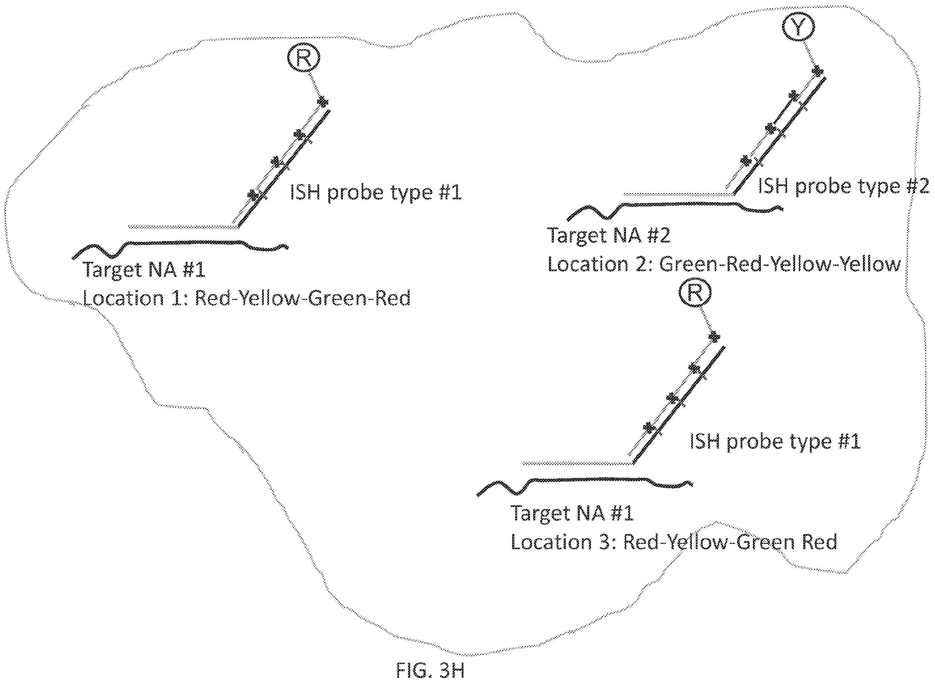

These steps are repeated until each of the attachment regions in each ISH probe has been bound by a reporter probe, and the identity of the detectable label of the reporter probe has been recorded. Thus, at the end of the method, a "linear order of detectable labels" will have been recorded at each location of interest. As shown in FIG. 3H, in this non-limiting example, the linear order of detectable labels at Location 1 and Location 3 was red-yellow-green-red and the linear order of detectable labels at Location 2 was green-red-yellow-yellow. Thus, given that red-yellow-green-red is specific to target nucleic acid #1 and green-red-yellow-yellow is specific to target nucleic acid #2, the method has allowed for the identification of two copies of target nucleic acid #1 in the biological sample, with one of the copies being present at Location 1 and one of the copies being present at Location 3, and the identification of one copy of target nucleic acid #2 at Location 2.

The above method can be multiplexed to detect any number of target nucleic acids and/or target proteins at any number of locations with a biological sample. In some aspects, the methods of the present disclosure can be used to determine the spatial abundance of at least about 10, or at least about 20, or at least about 30, or at least about 40, or at least about 50, or at least about 60, or at least about 70, or at least about 80, or at least about 90, or at least about 100, or at least about 110, or at least about 120, or at least about 130, or at least about 140, or at least about 150, or at least about 160, or at least about 170, or at least about 180, or at least about 190, or at least about 200, or at least about 210, or at least about 220, or at least about 240, or at least about 250, or at least about 260, or at least about 270, or at least about 280, or at least about 290, or at least about 300, or at least about 500, or at least about 1,000, or at least about 10,000, or at least about 100,000, or at least about 1,000,000 different species of target nucleic acids and/or target proteins within a biological sample.

Accordingly, the present disclosure provides a method of determining the abundance and spatial position of at least one target analyte in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target analyte in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes bound to a target analyte in the biological sample have been hybridized to a reporter probe comprising at least one detectable label, thereby determining the abundance and spatial position of the at least one target analyte in the biological sample based on the sequence in which the detectable labels were recorded.

Accordingly, the present disclosure provides a method of determining the abundance and spatial position of at least one target protein molecule in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target protein molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes bound to a target protein molecule in the biological sample have been hybridized to a reporter probe comprising at least one detectable label, thereby determining the abundance and spatial position of the at least one target protein molecule in the biological sample based on the sequence in which the detectable labels were recorded.

Accordingly, the present disclosure provides a method of determining the abundance and spatial position of at least one target nucleic acid molecule in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe hybridized to a target nucleic acid molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes hybridized to a target nucleic acid in the biological sample have been hybridized to a reporter probe comprising at least one detectable label, thereby determining the abundance and spatial position of the at least one target nucleic acid molecule in the biological sample based on the sequence in which the detectable labels were recorded.

Accordingly, the present disclosure provides a method of determining the abundance and spatial position of at least one target analyte in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target analyte in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment regions in the barcode domains of ISH probes bound to a target analyte in the biological sample have been hybridized to a reporter probe comprising at least one detectable label, thereby determining the abundance and spatial position of the at least one target analyte in the biological sample based on the sequence in which the detectable labels were recorded.

Accordingly, the present disclosure provides a method of determining the abundance and spatial position of at least one target protein molecule in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target protein molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment regions in the barcode domains of ISH probes bound to a target protein molecule in the biological sample have been hybridized to a reporter probe comprising at least one detectable label, thereby determining the abundance and spatial position of the at least one target protein molecule in the biological sample based on the sequence in which the detectable labels were recorded.

Accordingly, the present disclosure provides a method of determining the abundance and spatial position of at least one target nucleic acid molecule in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe hybridized to a target nucleic acid in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment regions in the barcode domains of ISH probes hybridized to a target nucleic acid in the biological sample have been hybridized to a reporter probe comprising at least one detectable label, thereby determining the abundance and spatial position of the at least one target nucleic acid molecule in the biological sample based on the sequence in which the detectable labels were recorded.

Accordingly, the present disclosure provides a method of determining the abundance and spatial position of at least two target analytes in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target analyte in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes bound to a target analyte in the biological sample have been hybridized to a reporter probe comprising at least one detectable label, thereby determining the abundance and spatial position of the at least two target analytes in the biological sample based on the sequence in which the detectable labels were recorded.

Accordingly, the present disclosure provides a method of determining the abundance and spatial position of at least two target protein molecules in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target protein molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes bound to a target protein molecule in the biological sample have been hybridized to a reporter probe comprising at least one detectable label, thereby determining the abundance and spatial position of the at least two target protein molecules in the biological sample based on the sequence in which the detectable labels were recorded.

Accordingly, the present disclosure provides a method of determining the abundance and spatial position of at least two target nucleic acid molecules in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe hybridized to a target nucleic acid molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region in the barcode domains of ISH probes hybridized to a target nucleic acid in the biological sample have been hybridized to a reporter probe comprising at least one detectable label, thereby determining the abundance and spatial position of the at least two target nucleic acid molecules in the biological sample based on the sequence in which the detectable labels were recorded.

Accordingly, the present disclosure provides a method of determining the abundance and spatial position of at least two target analytes in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target analyte in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment regions in the barcode domains of ISH probes bound to a target analyte in the biological sample have been hybridized to a reporter probe comprising at least one detectable label, thereby determining the abundance and spatial position of the at least two target analytes in the biological sample based on the sequence in which the detectable labels were recorded.

Accordingly, the present disclosure provides a method of determining the abundance and spatial position of at least two target protein molecules in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe bound to a target protein molecule in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment regions in the barcode domains of ISH probes bound to a target protein molecule in the biological sample have been hybridized to a reporter probe comprising at least one detectable label, thereby determining the abundance and spatial position of the at least two target protein molecules in the biological sample based on the sequence in which the detectable labels were recorded.

Accordingly, the present disclosure provides a method of determining the abundance and spatial position of at least two target nucleic acid molecules in a biological sample, the method comprising: a) contacting a biological sample prepared according to the sample preparation methods described herein with a plurality of reporter probes of the present disclosure, wherein each reporter probe comprises at least one detectable label, thereby hybridizing a reporter probe to an attachment region of a barcode domain of at least one ISH probe hybridized to a target nucleic acid in the biological sample; b) removing non-hybridized reporter probes from the biological sample; c) recording the identity and spatial position of the detectable labels of the hybridized reporter probes; d) removing the detectable labels of the hybridized reporter probes; and e) repeating steps (a)-(d) until each attachment region of the at least one, or at least two, or at least three, or at least four, or at least five, or at least six, or at least seven, or at least eight, or at least nine, or at least ten attachment region in the barcode domains of ISH probes hybridized to a target nucleic acid in the biological sample have been hybridized to a reporter probe comprising at least one detectable label, thereby determining the abundance and spatial position of the at least two target nucleic acid molecules in the biological sample based on the sequence in which the detectable labels were recorded.

In some aspects of the preceding methods, determining the abundance and spatial position of the target analyte(s) can comprise using the abundance and spatial position of said target analytes to define one or more regions of interest within the biological sample. In some aspects, after identifying the one or more regions of interest within the tissue sample, the nucleic acid probes (*i.e.* ISH probes) bound to the target analytes can be removed from the biological sample, the biological sample can be contacted again with at least one nucleic acid probe (*i.e.* ISH probe), and the imaging methods described above can be repeated only within the identified one or more regions of interest within the biological sample. Without wishing to be bound by theory, by identifying the one or more regions of interest within the biological sample, subsequent imaging rounds can be performed more quickly, as only certain areas of the tissue sample need to be interrogated as opposed to the entire tissue sample. In a non-limiting example, determining the abundance and spatial position of said target analytes to define one or more regions of interest can allow the skilled artisan performing the preceding methods to identify tumorous sections of a biological sample, and only these tumorous sections of the biological sample are then analyzed further in subsequent imaging cycles. This method of determining one or more regions of interests is referred to herein as morphology scanning.

In some aspects of the preceding methods, the biological sample can be a mounted biological sample prepared according to the sample processing methods described herein. In some aspects, the mounted biological sample is in a flow cell prepared using the sample processing methods described herein.

Any steps of the sample preparation methods described herein can be combined with any of the steps of the imaging methods described herein.

In some aspects of the preceding method, the method can further comprise, prior to step (a), pretreating the biological sample. In some aspects, pretreating the biological sample can comprise incubating the biological sample in a Sulfo-NHS acetate blocking solution. In some aspects, pretreating the biological sample can comprise washing the biological sample with Reporter Wash Buffer. In some aspects, pretreating the biological sample can comprise incubating the biological sample in an autofluorescence suppressor buffer. In some aspects, pretreating the biological sample can comprise illuminating the biological sample with blue and/or UV light, thereby quenching sample autofluorescence via photobleaching. In some aspects, any combination of UV and readout channel illumination can be used to quench sample autofluorescence via photobleaching.

In some aspects, pretreating the biological sample can comprise: i) incubating the biological sample in a Sulfo-NHS Acetate Blocking solution for about 15 minutes; and ii) washing the biological sample with Reporter Wash Buffer. In some aspects, pretreating the biological sample can comprise: i) incubating the biological sample in a Sulfo-NHS Acetate Blocking solution for about 15 minutes; ii) washing the biological sample with Reporter Wash Buffer; iii) incubating the biological sample in an autofluorescence suppressor buffer; and iv) washing the biological sample with Reporter Wash Buffer. In some aspects, pretreating the biological sample can comprise: i) incubating the biological sample in a Sulfo-NHS Acetate Blocking solution for about 15 minutes; ii) washing the biological sample with Reporter Wash Buffer; iii) incubating the biological sample in an autofluorescence suppressor buffer and/or illuminating the biological sample with blue and/or UV light, thereby quenching sample autofluorescence via photobleaching; and iv) washing the biological sample with Reporter Wash Buffer.

In some aspects, washing the biological sample can comprise washing the biological sample with at least about 1000 ml of Reporter Wash Buffer.

In some aspects, Reporter Wash Buffer can comprise a solution of 0.5% Tween-20 in 1x SSPE solution.

In some aspects, an autofluorescence suppressor buffer can comprise any buffer that decreases autofluorescence of tissue samples, as would be appreciated by the skilled artisan. A non-limiting example of an autofluorescence suppressor buffer is TrueBlack Background Suppressor Solution (available from Biotium, Inc., Fremont, CA),

As would be appreciated by the skilled artisan, 20x SSPE buffer comprises 0.02M EDTA and 2.98M NaCL in 0.2M phosphate buffer pH 7.4.

In some aspects, a Sulfo-NHS Acetate Blocking Solution can comprise a solution of 100 mM Sulfo-NHS acetate and 100 mM Sodium Phosphate pH 8.0.

In some aspects of the preceding method, contacting the biological sample with a plurality of reporter probes of the present disclosure can comprise incubating the biological sample with a solution, wherein the solution comprises at least one species of reporter probe at a concentration of 5 nM, 8.75x SSPE solution, 0.5% Tween-20 and 0.1% RNase inhibitor in DEPC-treated water for at least about 15 minutes. In some aspects, of the preceding method, contacting the biological sample with a plurality of reporter probes of the present disclosure can comprise incubating the biological sample with a solution for about 15 minutes, wherein the solution comprises more than one species of reporter probe, wherein at least one species of reporter probe is present at a concentration of 5 nM, and the solution further comprises 8.75x SSPE solution, 0.5% Tween-20 and 0.1% RNase inhibitor.

In some aspects of the preceding method, contacting the biological sample with a plurality of reporter probes of the present disclosure can comprise incubating the biological sample with a solution, wherein the solution comprises at least one species of reporter probe at a concentration of 5 nM, 8.75x SSPE solution, 0.5% Tween-20 and optionally 0.1% RNase inhibitor in DEPC-treated water for at least about 15 minutes. In some aspects of the preceding method, contacting the biological sample with a plurality of reporter probes of the present disclosure can comprise incubating the biological sample with a solution for about 15 minutes, wherein the solution comprises more than one species of reporter probe, wherein at least one species of reporter probe is present at a concentration of 5 nM, and the solution further comprises 8.75x SSPE solution, 0.5% Tween-20 and optionally 0.1% RNase inhibitor.

In some aspects of the preceding method, removing non-hybridized reporter probes from the biological sample can comprise washing the biological sample with Reporter Wash Buffer. In some aspects, removing non-hybridized reporter probes from the biological sample can comprise washing the biological sample with at least about 2000 mL of Reporter Wash Buffer.

In some aspects of the preceding method, recording the identity and spatial position within the biological sample of the detectable label of the hybridized reporter probes can comprise: i) immersing the biological sample in Imaging Buffer; and ii) imaging the biological sample to record the identity and spatial position of the detectable labels of the hybridized reporter probes.

In some aspects, the Imaging Buffer can match the refractive index of water and allow for imaging of fiduicals and reporter probes without bleaching or reduction of fluorescent signal. In a non-limiting example, Imaging Buffer can allow for up to 4000 rounds of imaging per location on the tissue without bleaching or reduction of fluorescent signal

Imaging Buffers of the present disclosure can comprise one or more of the following: pyranose oxidase, catalase, glucose oxidase, tris (2-carboxyethyl) phosphine (TCEP), dithiothreitol (DTT), 2-mercaptoethanol (BME), p-phenylenediamine (PPD), n-propyl gallate (NPG), 1,4-diazobicyclo[2,2,2]-octane (DABCO), ascorbic acid, 3-carboxy-proxyl, 4-hydroxy-2,2,6,6-tetramethylpiperidin-1-oxyl (TEMPOL), N-tert-butyl-α-phenyinitrone, N-tert-butyl-α-(2-sulfophenyl)nitrone, 5,5-dimethyl-1-pyrroline N-oxide, ethyl 4,4,4-trifluorobutyrate, 4-hydrazonomethyl-1-hydroxy-2,2,5,5-tetramethyl-3-imidazoline-3-oxide, α-(4-pyridyl N-oxide)-N-tert-butylnitrone, silver diethyldithiocarbamate, sodium diethyldithiocarbamate trihydrate, 3,3,5,5-tetramethyl-1-pyrroline N-oxide, 1,3,5-tri-tert-butyl-2-nitrosobenzene, 2-(5,5-Dimethyl-2-oxo-2λ5-[1,3,2]dioxaphosphinan-2-yl)-2-methyl-3,4-dihydro-2H-pyrrole 1-oxide(CYPMPO), vitamin E, -hydroxy-2,5,7,8-tetramethylchroman-2-carboxylic acid (Trolox), N-acetyl-L-cysteine, 4-aminobenzohydrazide (myeloperoxidase Inhibitor-I), balsalazide disodium salt hydrate, bilirubin, N-tert-butyl-α-phenylnitrone, caffeic acid, β-carotene, catechin gallate, chlorogenic acid, chlorophyllin sodium, p-coumaric acid, delphinidin chloride, 5-O-(trans-3,4-Dihydroxycinnamoyl)-D-quinic acid, DL-α-lipoic Acid, ellagic acid, 2,4-dihydro-5-methyl-2-phenyl-3H-pyrazol-3-one(MCI-186), (-)-epicatechin, (---)-Epicatechin gallate, EUK-8, trans-ferulic acid, 4-(5-Fluoro-1H-indol-3-yl)butanamide, MPO Inhibitor II (Myeloperoxidase Inhibitor-II), Fe(III)tetrakis (1-methyl-4-pyridyl) porphyrin pentachlorideporphyrin pentachloride, Fe(III)5,10,15,20-tetrakis(4-sulfonatophenyl)porphyrinato chloride, fucoxanthin carotenoid antioxidant, gallic acid, (-)-gallocatechin, ginkgolide , glutathione monoethyl ester, glutathione free acid, hesperidin, 3-hydroxytyrosol, 7-hydroxy-3-(4-methoxyphenyl)chromen-4-one (formononetin), kaempferol, linoleic acid, (±)-α-lipoic acid, luteolin, lycopene, L-lysine , Mn(III)tetrakis(4-benzoic acid)porphyrin chloride (MnTBAP), meso-Tetra(N-methyl-4-pyridyl)porphine tetratosylate salt (TMPyP), oleic acid, resveratrol, rutin hydrate, seleno-L-methionine, se-(Methyl)selenocysteine, sodium selenite, taxifolin hydrate, (+)-α-tocopherol, and xanthophyll.

In some aspects, Imaging Buffer can comprise a solution of 98% Low Salt Imaging Buffer, 1% Protocatechuic Acid (PCA) and 1% Protocatechuate dioxygenase (PCD). In some aspects, Low Salt Imaging Buffer can comprise a solution of 1 M Tris-HCL pH 7.5, 5M Sodium Chloride and 0.5% Tween-20 in DEPC-treated water.

In some aspects of the preceding method, removing the detectable labels of the hybridized reporter probes can comprise: i) illuminating the biological sample with UV light sufficient to cleave photocleavable linker moieties in the hybridized reporter probes, and ii) washing the biological sample with Reporter Wash Buffer. In some aspects of the preceding method, removing the detectable labels of the hybridized reporter probes can comprise: i) illuminating the biological sample with UV light sufficient to cleave photocleavable linker moieties in the hybridized reporter probes; ii) washing the biological sample with Reporter Wash Buffer; iii) immersing the biological sample in Imaging Buffer, and iv) imaging the sample to ensure that there are no remaining detectable labels. In some aspects, washing the biological sample with Reporter Wash Buffer can comprise washing the biological sample with at least about 2000 mL of Reporter Wash buffer.

In some aspects of the preceding method, removing the detectable labels of the hybridized reporter probes can comprise: i) illuminating the biological sample with UV light sufficient to cleave photocleavable linker moieties in the hybridized reporter probes; and ii) washing the biological sample with Strip Wash Buffer. In some aspects of the preceding method, removing the detectable labels of the hybridized reporter probes can comprise: i) illuminating the biological sample with UV light sufficient to cleave photocleavable linker moieties in the hybridized reporter probes; ii) washing the biological sample with Strip Wash Buffer; iii) immersing the biological sample in Imaging Buffer; and iv) imaging the sample to ensure that there are no remaining detectable labels. In some aspects, washing the biological sample with Strip Wash Buffer can comprise washing the biological sample with at least about 2000 mL of Strip Wash buffer.

Without wishing to be bound by theory, the combination of illuminating the biological sample with UV light sufficient to cleave photocleavable linker moieties in the hybridized reporter probes and washing the biological sample with Strip Wash Buffer unexpectedly results in more efficient and complete removal of all fluorescent labels, thereby removing possible fluorescence contamination from future imagine cycles.

In some aspects, washing the biological sample with Reporter Wash Buffer can comprise washing at a flow rate of about 0.20 ml/min to about 0.55 ml/min, or about 0.60 ml/min to about 0.90 ml/min, or about 0.65 ml/min to about 0.85 ml/min, or about 0.70 ml/min to about 0.85 ml/min, or about 0.75 ml/min. In some aspects, washing the biological sample with Reporter Wash Buffer can comprise washing at a flow rate of about 0.75 ml/min. In some aspects, washing the biological sample with Reporter Wash Buffer can comprise washing at a flow rate of 0.20 ml/min to 0.55 ml/min, 0.60 ml/min to 0.90 ml/min, or 0.65 ml/min to 0.85 ml/min, or 0.70 ml/min to 0.85 ml/min, or 0.75 ml/min. In some aspects, washing the biological sample with Reporter Wash Buffer can comprise washing at a flow rate of 0.75 ml/min.

In some aspects, washing the biological sample with Reporter Wash Buffer can comprise washing such that there is a sheer stress at the sample plane of about 0.01 dyn/cm² to about 20 dyn/cm², or about 0.01 dyn/cm² to about 100 dyn/cm², or about 8.89 dyn/cm², or about 9 dyn/cm². In some aspects, washing the biological sample with Reporter Wash Buffer can comprise washing such that there is a sheer stress at the sample plane of about 8.89 dyn/cm². In some aspects, washing the biological sample with Reporter Wash Buffer can comprise washing such that there is a sheer stress at the sample plane of 0.01 dyn/cm² to 20 dyn/cm², or 0.01 dyn/cm² to 100 dyn/cm², or 8.89 dyn/cm², or 9 dyn/cm². In some aspects, washing the biological sample with Reporter Wash Buffer can comprise washing such that there is a sheer stress at the sample plane of 8.89 dyn/cm².

In some aspects, washing the biological sample with Strip Wash Buffer can comprise washing at a flow rate of about 0.25 ml/min to about 0.55 ml/min, or about 0.3 ml/min to about 0.5 ml/min, or about 0.35 ml/min to about 0.45 ml/min, or about 0.4 ml/min. In some aspects, washing the biological sample with Strip Wash Buffer can comprise washing at a flow rate of about 0.4 ml/min. In some aspects, washing the biological sample with Strip Wash Buffer can comprise washing at a flow rate of 0.25 ml/min to 0.55 ml/min, or 0.3 ml/min to 0.5 ml/min, or 0.35 ml/min to 0.45 ml/min, or 0.4 ml/min. In some aspects, washing the biological sample with Strip Wash Buffer can comprise washing at a flow rate of 0.4 ml/min.

In some aspects, washing the biological sample with Strip Wash Buffer can comprise washing such that there is a sheer stress at the sample plane of about 0.01 dyn/cm² to about 20 dyn/cm², or about 0.01 dyn/cm² to about 100 dyn/cm², or about 8.89 dyn/cm², or about 9 dyn/cm². In some aspects, washing the biological sample with Strip Wash Buffer can comprise washing such that there is a sheer stress at the sample plane of about 8.89 dyn/cm². In some aspects, washing the biological sample with Strip Wash Buffer can comprise washing such that there is a sheer stress at the sample plane of 0.01 dyn/cm² to 20 dyn/cm², or 0.01 dyn/cm² to 100 dyn/cm², or 8.89 dyn/cm², or 9 dyn/cm². In some aspects, washing the biological sample with Strip Wash Buffer can comprise washing such that there is a sheer stress at the sample plane of 8.89 dyn/cm².

In some aspects, any of the methods of the present disclosure can further comprise morphology scanning of the biological sample. In some aspects, morphology scanning can be used to determine one or more regions of interest to be imaged. In some aspects, morphology scanning can be used to identify specific features of the biological sample (e.g. tumorous cells, healthy cells, tumor margins, cellular membranes, cellular nuclei, one or more cellular organelles, vasculature, or any other features known in the art by the skilled artisan). In some aspects, the specific features of the biological sample can be correlated with the abundance and spatial position of target analytes measured using the methods of the present disclosure. In some aspects, morphology scanning can be used to determine the boundaries of individual cells within the biological sample. The determination of the boundaries of individual cells is referred to herein as "cell segmentation".

In some aspects of the preceding method, the method can further comprise staining the biological sample with a membrane specific-fluorescent staining solution and imaging the biological sample to identify the spatial location of cellular membranes within the sample. This staining can be performed at any step in the protocol, *e*.*g*. before contacting the mounted biological sample with at least one nucleic acid probe, contacting a biological sample prepared according to the sample preparation methods described herein with a plurality of reporter probes of the present disclosure, after step (e), etc.

In some aspects of the preceding method, the method can further comprise staining the biological sample with a nuclear-specific fluorescent staining solution and imaging the biological sample to identify the spatial location of cellular nuclei in the sample. This staining can be performed at any step in the protocol, *e.g.* before contacting the mounted biological sample with at least one nucleic acid probe, contacting a biological sample prepared according to the sample preparation methods described herein with a plurality of reporter probes of the present disclosure, after step (e), etc.

In some aspects of the preceding method, the method can further comprise, after step (e), staining the biological sample with a membrane specific-fluorescent staining solution and imaging the biological sample to identify the spatial location of cellular membranes within the sample.

In some aspects of the preceding method, the method can further comprise, after step (e), staining the biological sample with a nuclear-specific fluorescent staining solution and imaging the biological sample to identify the spatial location of cellular nuclei in the sample.

In some aspects, membrane and/or nuclear stains are used to perform morphology scanning on the biological sample. Accordingly, in aspects wherein the membrane and/or nuclear stains are performed before contacting the biological sample with at least one nucleic acid probe or a plurality of reporter probes, the membrane and/or nuclear stains can be used to determine one or more regions of interest to be imaged during determination of the abundance and spatial position of target analytes (e.g. target nucleic acid molecules, target protein molecules, etc.). Without wishing to be bound by theory, by determining the region to be imaged using the membrane and/or nuclear stains, the total time to run imaging experiments can be decreased by imaging only particular regions of interest, as the total duration of an experiment increases as more areas of the biological sample are imaged. Morphology scanning using membrane and/or nuclear stains can be performed before or after any steps of any of the methods of the present disclosure and can also be repeated multiple times.

In some aspects, performing morphology scanning on the biological sample can comprise incubating the biological sample with immunostaining blocking buffer. In some aspects, immunostaining blocking buffer can comprise Buffer W. In some aspects, immunostaining blocking buffer can comprise about 2% to about 5% BSA/BCS and about 0.5% Tween20 in about 8.75x SSPE. In some aspects, immunostaining blocking buffer can comprise 2% to 5% BSA/BCS and 0.5% Tween20 in 8.75x SSPE.

In some aspects, performing morphology scanning on a biological sample can comprise contacting the biological sample with at least one probe. In some aspects, the at least one probe can be an ISH probe of the present disclosure. In some aspects, the at least one probe can be an antibody conjugated to a barcode domain disclosed herein. In some aspects, the at least one probe can be a lectin targeting morphology protein conjugated to a barcode domain disclosed herein.

In some aspects, after contacting the biological sample with at least one probe, morphology scanning can continue by washing the biological sample with PBS to remove any unbound probes.

In some aspects, after washing the biological sample with PBS to remove any unbound probes, morphology scanning can continue with visualizing the probes (e.g. through the binding of one or more reporter probes to the barcode domains). Visualizing the probes can comprise: i) contacting the biological sample with reporter probes described herein that hybridize to the barcode domains of the ISH probes; ii) washing the biological sample with Reporter Wash Buffer; iii) incubating the biological sample with imagine buffer; iv) recording the identity and spatial position of the detectable labels of the hybridized reporter probes. Visualizing the probes can further comprise removing the detectable labels of the hybridized reporter probes using similar steps described herein, and repeating steps (i) - (iv) until each attachment position in the barcode domains have been hybridized to a reporter probe.

In some aspects of the preceding method, the method can further comprise, after step (e): f) washing the biological sample with Strip Wash Buffer; g) immersing the biological sample in Imaging Buffer; h) imaging the biological sample to ensure that there are no remaining detectable labels; i) incubating the biological sample with Membrane Stain Blocking Solution; j) incubating the biological sample with Membrane Stain solution; k) washing the biological sample Reporter Wash Buffer; l) immersing the biological sample in Imaging Buffer; m) imaging the biological sample to record the spatial position of the cellular membranes in the biological solution; n) incubating the sample with Nuclear Stain Solution; o) washing the biological sample with Reporter Wash Buffer; p) immersing the sample in Imaging Buffer; and q) imaging the biological sample to record the spatial position of cellular nuclei in the sample.

In some aspects of the preceding method, the method can further comprise, after step (e): f) washing the biological sample with Strip Wash Buffer; g) immersing the biological sample in Imaging Buffer; h) imaging the biological sample to ensure that there are no remaining detectable labels; i) incubating the sample with Nuclear Stain Solution; j) washing the biological sample with Reporter Wash Buffer; k) immersing the sample in Imaging Buffer; and l) imaging the biological sample to record the spatial position of cellular nuclei in the sample; m) incubating the biological sample with Membrane Stain Blocking Solution; n) incubating the biological sample with Membrane Stain solution; o) washing the biological sample Reporter Wash Buffer; p) immersing the biological sample in Imaging Buffer; q) imaging the biological sample to record the spatial position of the cellular membranes in the biological solution.

In some aspects of the preceding method, the method can further comprise, before or after any step: i) washing the biological sample with Strip Wash Buffer; ii) immersing the biological sample in Imaging Buffer; iii) imaging the biological sample to ensure that there are no remaining detectable labels; iv) incubating the biological sample with Membrane Stain Blocking Solution; v) incubating the biological sample with Membrane Stain solution; vi) washing the biological sample Reporter Wash Buffer; vii) immersing the biological sample in Imaging Buffer; viii) imaging the biological sample to record the spatial position of the cellular membranes in the biological solution; ix) incubating the sample with Nuclear Stain Solution; x) washing the biological sample with Reporter Wash Buffer; xi) immersing the sample in Imaging Buffer; and xii) imaging the biological sample to record the spatial position of cellular nuclei in the sample

In some aspects of the preceding method, the method can further comprise, before or after any step: i) washing the biological sample with Strip Wash Buffer; ii) immersing the biological sample in Imaging Buffer; iii) imaging the biological sample to ensure that there are no remaining detectable labels; iv) incubating the sample with Nuclear Stain Solution; v) washing the biological sample with Reporter Wash Buffer; vi) immersing the sample in Imaging Buffer; and vii) imaging the biological sample to record the spatial position of cellular nuclei in the sample; viii) incubating the biological sample with Membrane Stain Blocking Solution; ix) incubating the biological sample with Membrane Stain solution; x) washing the biological sample Reporter Wash Buffer; xi) immersing the biological sample in Imaging Buffer; xii) imaging the biological sample to record the spatial position of the cellular membranes in the biological solution.

In some aspects, the biological sample can be incubated with the Membrane Stain Blocking Solution for at least about 30 minutes.

In some aspects, the biological sample can be incubated with the Membrane Stain solution for at least about 60 minutes.

In some aspects, the biological sample can be incubated with the Nuclear Stain solution for at least about 5 minutes. In some aspects, nuclear stain solution can comprise 4',6-diamidino-2-phenylindole (DAPI) or DAPI, dilactate. In some aspects, Nuclear Stain solution can comprise about 100 nM to about 500 nM DAPI diluted in PBS. In some aspects, nuclear stain solution can comprise about 300 nM DAPI diluted in PBS.

In some aspects, Membrane Stain Solution can comprise a solution of 5% NaN₃ and 1% DAPI in Buffer W, further comprising at least one of a fluorescently labeled anti-CD298 antibody, a fluorescently labeled anti-CD3 antibody, a fluorescently labeled anti-CD20 antibody, and a fluorescently labeled anti-PanCK antibody.

In some aspects, Membrane Stain Solution can comprise a solution of Membrane Stain Blocking Solution, further comprising at least one of a fluorescently labeled anti-CD298 antibody, a fluorescently labeled anti-B2M antibody, a fluorescently labeled anti-CD3 antibody, a fluorescently labeled anti-CD20 antibody, a fluorescently labeled anti-PanCK antibody, a fluorescently labeled anti-CD3 antibody, a fluorescently labeled anti-Histone H3 antibody, a fluorescently labeled wheat germ agglutinin protein, and a fluorescently labeled concanavalin A protein.

In some aspects, Strip Wash Buffer can comprise a solution of 0.0033x SSPE buffer and 0.5% Tween-20. In some aspects, Strip Wash Buffer can comprise a solution of about 0.0033x SSPE buffer and about 0.5% Tween-20.

In some aspects, Strip Wash Buffer can comprise a solution of 0.0033x SSPE buffer, 0.1% ProClin 950 and 0.5% Tween-20. In some aspects, Strip Wash Buffer can comprise a solution of about 0.0033x SSPE buffer, about 0.1% ProClin 950 and about 0.5% Tween-20.

In some aspects, Membrane Stain Blocking Solution can comprise a solution of 0.5% NaN₃ and 1% 4',6-diamidino-2-phenylindole (DAPI) in Buffer W. In some aspects, Membrane Stain Blocking Solution can comprise a solution of about 0.5% NaN₃ and about 1% 4'.6-diamidino-2-phenylindole (DAPI) in Buffer W.

In some aspects, Membrane Stain Blocking Solution can comprise a solution of 0.5% NaN₃ and 2 µg/mL 4',6-diamidino-2-phenylindole (DAPI) in Buffer W. In some aspects, Membrane Stain Blocking Solution can comprise a solution of about 0.5% NaN₃ and about 2 µg/mL 4',6-diamidino-2-phenylindole (DAPI) in Buffer W.

In some aspects, Buffer W can comprise at least one of bovine calf serum (BCS), sodium azide (NaN₃), dextran sulfate and ssDNA. In some aspects, Buffer W can comprise a combination of BCS, NaN₃, dextran sulfate and ssDNA. In some aspects, the concentration of dextran sulfate in Buffer W can be about 0.001% to about 0.1%, or about 0.005% to about 0.05%. In some aspects, the concentration of dextran sulfate in Buffer W can be about 0.01%. In some aspects, the concentration of ssDNA in Buffer W can be about 0.01 mg/ml to about 1 mg/ml, or about 0.05 mg/ml to about 0.5 mg/ml. In some aspects, the concentration of ssDNA in Buffer W can be about 0.1 mg/ml.

In some aspects, performing morphology scanning on the biological sample can comprise: i) contacting the biological sample with at least one ISH probe, wherein the at least one ISH probe comprises a unique target binding domain that binds to a target analyte in the biological sample and a unique barcode domain specific for the target analyte, wherein the barcode domain comprises at least one attachment position, ii) contacting the prepared biological sample with a plurality of reporter probes, wherein each reporter probe comprises at least one detectable label, thereby hybridizing a reporter probe to an attachment region of a barcode domain of at least one ISH probe hybridized to a target analyte in the biological sample; iii) removing non-hybridized reporter probes from the biological sample; iv) recording the identity and spatial position of the detectable labels of the hybridized reporter probes; v) removing the detectable labels of the hybridized reporter probes, and optionally vi) repeating steps (ii)-(v) until each attachment position in the barcode domains of ISH probes hybridized to a target analyte in the biological have been hybridized to a reporter probe comprising at least one detectable label, thereby determining the abundance and/or spatial position of the at least two target analytes in the biological sample based on the sequence in which the detectable labels were recorded, thereby determining one or more regions of interest. In some aspects of the preceding method, the target binding domain can comprise an antibody. In some aspects of the preceding method, the target binding domain can comprise a lectin protein. In some aspects of the preceding methods, the barcode domains comprise one attachment position.

In some aspects of the preceding method, fiducial markers added to the biological sample can be used to focus the biological sample using methods standard in the art, as would be appreciated by the skilled artisan. Specifically, the fiducial markers can be used to determine the best z-position for imaging a particular location within the biological sample. Additionally,

### Kits

The present disclosure provides kits for use in the methods of the present disclosure.

In some aspects, a kit of the present disclosure can comprise any of the buffers and/or solutions described herein.

In some aspects, a kit of the present disclosure can comprise a plurality of ISH probes of the present disclosure.

In some aspects, a kit of the present disclosure can comprise a plurality of reporter probes of the present disclosure.

In some aspects, a kit of the present disclosure can comprise an apparatus suitable for use in the methods of the present disclosure.

### Exemplary Embodiments:

Embodiment 1. A method of preparing a biological sample for fluorescent imaging, the method comprising:
a) mounting a biological sample onto a functionalized microscope slide thereby producing a mounted biological sample, wherein the biological sample is a formalin fixed paraffin embedded (FFPE) microtome section;
b) baking the mounted biological sample;
c) deparaffinizing the mounted biological sample;
d) performing a target retrieval reaction on the mounted biological sample;
e) permeabilizing the mounted biological sample;
f) applying at least one fiducial marker to the mounted biological sample;
g) fixing the mounted biological sample;
h) contacting the mounted biological sample with at least one nucleic acid probe;
i) washing the mounted biological sample.

Embodiment 2. The method of embodiment 1, further comprising after step (j), assembling the mounted biological sample into a flow cell.

Embodiment 3. The method of any of the preceding embodiments, wherein the functionalized microscope slide is a (3-Aminopropyl)trimethoxysilane (APTMS)-functionalized microscope slide.

Embodiment 4. The method of any of the preceding embodiments, wherein the biological sample is an FFPE microtome section of a human tissue sample.

Embodiment 5. The method of any of the preceding embodiments, wherein step (b) comprises baking the mounted biological sample at about 60°C for about 1 hour.

Embodiment 6. The method of any of the preceding embodiments, wherein step (c) comprises:
i) incubating the mounted biological sample in a first solution of xylene for about 5 minutes,
ii) incubating the mounted biological sample in a second solution of xylene for about 5 minutes,
iii) incubating the mounted biological sample in a first 100% ethanol solution for about 2 minutes,
iv) incubating the mounted biological sample in the second 100% ethanol solution for about 2 minutes; and
v) drying the mounted biological sample at about 60°C for about 5 minutes.

Embodiment 7. The method of any of the preceding embodiments, wherein step (d) comprises:
i) incubating the mounted biological sample in target retrieval solution at about 100°C;
ii) incubating the mounted biological sample in DEPC-treated water for about 15 seconds;
iii) incubating the mounted biological sample in a solution of 100% ethanol for about 3 minutes; and
iv) drying the mounted biological sample.

Embodiment 8. The method of embodiment 7, wherein the mounted biological sample is incubated in the target retrieval solution for a time period as put forth in Table 1.

Embodiment 9. The method of embodiment 7 or embodiment 8, wherein the target retrieval solution comprises TRIS and EDTA solution and has a pH of about 9.

Embodiment 10. The method of any of the preceding embodiments, wherein step (e) comprises:
i) incubating the mounted biological sample in a proteinase K solution at about 40°C, wherein the concentration of proteinase K in the proteinase K solution is about 1 µg/mL ;
ii) washing the biological sample with a first aliquot of DEPC-treated water; and
iii) washing the biological sample with a second aliquot of DEPC-treated water.

Embodiment 11. The method of embodiment 10, wherein the mounted biological sample is incubated in the proteinase K solution for a time period as put forth in Table 2.

Embodiment 12. The method of any of the preceding embodiments, wherein step (f) comprises:
i) incubating the mounted biological sample in a solution comprising at least one fiducial marker for about 5 minutes at about room temperature, wherein the solution comprising at least one fiducial marker is a solution comprising carboxylated microspheres in red, blue and/or green at a concentration of about 0.001% and non-carboxylated FNDs at a concentration of about 0.00045% in 2x SSC solution; and
ii) washing the mounted biological sample with 1x PBS.

Embodiment 13. The method of any of the preceding embodiments, wherein step (g) comprises
i) incubating the mounted biological sample in a 10% NBF for about 5 minutes;
ii) incubating the mounted biological sample in a first tris glycine buffered solution for about 5 minutes;
iii) incubating the mounted biological sample in a second tris glycine buffered solution for about 5 minutes; and
iv) incubating the mounted biological sample in 1x PBS for about 5 minutes.

Embodiment 14. The method of any of the preceding embodiments, wherein step (h) comprises:
incubating the mounted biological sample with a solution comprising a plurality of ISH probes for about 16 to about 18 hours at about 37°C, thereby hybridizing at least one ISH probe to a target nucleic acid in the biological sample,
wherein the solution comprises at least two species of ISH probe, wherein at least one species of ISH probe is present at a concentration of about 200 nM,
wherein at least one species of ISH probe comprises a unique target binding domain that hybridizes to one of at least two target nucleic acids and a unique barcode domain specific for the target nucleic acid, wherein the barcode domain comprises at least four attachment positions.

Embodiment 15. The method of embodiment 14,
wherein the target binding domain is a single-stranded polynucleotide comprising a nucleic acid sequence that is complementary to a target nucleic acid,
wherein the target binding domain is about 35 to about 40 nucleotides in length, and wherein the target binding domain comprises D-DNA, and
wherein the barcode domain is a single-stranded polynucleotide comprising at least four attachment regions,
   wherein each attachment region comprises about one attachment sequence,
   wherein each of the attachment sequences is about 14 nucleotides in length,
   and wherein the sequences of each of the attachment sequences are different,
and wherein the barcode domain comprises L-DNA.

Embodiment 16. The method of any of the preceding embodiments, wherein step (i) comprises:
i) incubating the mounted biological sample with first 2x SSC solution;
ii) incubating the mounted biological sample in a first formamide solution;
iii) incubating the mounted biological sample with a second formamide solution;
iv) incubating the mounted biological sample with a second 2x SSC solution; and
v) incubating the mounted biological sample with a third 2x SSC solution.

Embodiment 17. A method of determining the abundance and spatial position of at least two target nucleic acid molecules in a biological sample, the method comprising:
a) contacting the biological sample prepared according to any one of the preceding embodiments with a plurality of reporter probes of the present disclosure, wherein each reporter probe comprises at least one detectable label, thereby hybridizing a reporter probe to an attachment region of a barcode domain of at least one ISH probe hybridized to a target nucleic acid in the biological sample;
b) removing non-hybridized reporter probes from the biological sample;
c) recording the identity and spatial position of the detectable labels of the hybridized reporter probes;
d) removing the detectable labels of the hybridized reporter probes; and
e) repeating steps (a)-(d) until each attachment position of the at least four attachment positions in the barcode domains of ISH probes hybridized to a target nucleic acid in the biological sample have been hybridized to a reporter probe comprising at least one detectable label,
thereby determining the abundance and spatial position of the at least two target nucleic acid molecules in the biological sample based on the sequence in which the detectable labels were recorded.

Embodiment 18. The method of embodiment 17, wherein the reporter probes comprise:
a primary nucleic acid molecule comprising a first domain, a second domain and a photocleavable linker located between the first domain and the second domain,
wherein the second domain of the primary nucleic acid molecule is hybridized to about six secondary nucleic acid molecules,
wherein each secondary nucleic acid molecule comprises a first domain, a second domain and a photocleavable linker located between the first domain and the second domain,
wherein the first domain of each of the secondary nucleic acid molecules is hybridized to the second domain of the primary nucleic acid molecule,
wherein the second domain of each of the secondary nucleic acid molecules is hybridized to about five tertiary nucleic acid molecules,
wherein each of the tertiary nucleic acid molecules comprise at least one detectable label, and
wherein the primary nucleic acid molecule, the secondary nucleic acid molecules, and the tertiary nucleic acid molecules comprise L-DNA.

Embodiment 19. The method of embodiment 17 or 18, wherein the at least one detectable label is a fluorescent moiety.

Embodiment 20. The method of any one of embodiments 17, 18 or 19, the method further comprising prior to step (a):
pretreating the biological sample by:
i) incubating the biological sample in a Sulfo-NHS Acetate Blocking solution for about 15 minutes;
ii) washing the biological sample with Reporter Wash Buffer;
iii) incubating the biological sample in autofluorescence suppressor buffer; and
iv) washing the biological sample with Reporter Wash Buffer.

Embodiment 21. The method of any one of embodiments 17, 18, 19 or 20, wherein step (a) comprises incubating the biological sample with a solution comprising the reporter probes at a concentration of 5 nM, 8.75x SSPE solution, 0.5% Tween-20 and 0.1% RNase inhibitor in DEPC-treated water for at least about 15 minutes.

22. The method of any one of embodiments 17, 18, 19, 20 or 21, wherein step (b) comprises washing the biological sample with Reporter Wash Buffer.

23. The method of any one of embodiments 17, 18, 19, 20, 21 or 22, wherein step (c) comprises: i) immersing the biological sample in Imaging Buffer; and ii) imaging the biological sample to record the identity and spatial position of the detectable labels of the hybridized reporter probes.

24. The method of any one of embodiments 17, 18, 19, 20, 21, 22 or 23, wherein step (d) comprises:
i) illuminating the biological sample with UV light sufficient to cleave photocleavable linker moieties in the hybridized reporter probes;
ii) washing the biological sample with Reporter Wash Buffer;
iii) immersing the biological sample in Imaging Buffer; and
iv) imaging the sample to ensure that there are no remaining detectable labels.

Embodiment 25. The method of any one of embodiments 17, 18, 19, 20, 21, 22, 23 or 24, the method further comprising, after step (e), staining the biological sample with a membrane specific-fluorescent staining solution and imaging the biological sample to identify the spatial location of cellular membranes within the sample.

Embodiment 26. The method of any one of embodiments 17, 18, 19, 20, 21, 22, 23, 24 or 25, the method further comprising, after step (e), staining the biological sample with a nuclear-specific fluorescent staining solution and imaging the biological sample to identify the spatial location of cellular nuclei in the sample.

### Examples

### Example 1a

The following non-limiting example describes a sample preparation protocol for use in the methods of the present disclosure.

First, a 5 µm FFPE microtome section of tissue is mounted onto a first microscope slide functionalized with 0.5% (3-Aminopropyl)trimethoxysilane. The first microscope slide was functionalized using methods standard in the art, as would be appreciated by the skilled artisan. As part of the sample preparation, the first microscope slide will serve as one surface of the flow cell that is to be assembled.

The slides are then baked at 60°C oven for 1 hour. Following baking, the paraffin on the slides is removed using xylene. Next, the previous paraformaldehyde fixation is undone by heating the slides at 100°C for 8 minutes in a target retrieval solution (*e*.*g*. 10mM Tris, 1mM EDTA at pH 9.0). The length of heating can be adjusted for the different sample type (*e.g.* tissue, cell pellet, etc.). In a non-limiting example, the slides can be heated for 8 minutes in the case of cell pellets.

After heating, a mixture of 0.001% 200nm carboxylated microsphere fiducials in red, blue, and green and 0.00045% non-carboxylated fluorescent nano-diamonds (FND) are applied to the tissue and incubated for 5 minutes before washed off with 1X Phosphate Buffered Solution (PBS).

Without wishing to be bound by theory, the mixture of fiducials and FNDs are used in subsequent imaging steps for autofocusing and image registration.

Following addition of fiducials and FNDs, the tissue sample is then post-fixed in 10% Neutral Buffered Formalin (NBF) for 5 minutes. Without wishing to be bound by theory, this post-fixing step is used to preserve the morphology of the tissue sample. The NBF is then neutralized with Tris Glycine buffer for 10 minutes and washed with 1X PBS.

In-situ hybridization (ISH) probe of the present disclosure are then denatured at 95°C for 2 minutes and crash cooled before being applied to the tissue sample at a 0.5 nM per probe concentration in a hybridization mix that contains Buffer R solution (Stock solution: 3.125% Dextran Sulfate, 0.25% BSA, 0.125mg/mL ssDNA, 2.5X SSC, 50% formamide; Working concentration: 2.5% Dextran Sulfate, 0.2% BSA, 0.1 mg/mL ssDNA, 2X SSC, 40% formamide) and RNAse inhibitor. The tissue sample is incubated with the ISH probes for 16-18 hours at 37° C.

Following incubation with the ISH probes, slides are then briefly dipped into 2x saline-sodium citrate (SSC) with 0.1% Tween-20 solution then incubated in 2 changes of 50% formamide and 2x SSC solution for at least 50 minutes to wash off excess, unbound ISH probes. Slides are then optionally dehydrated in an ethanol gradient using methods standard in the art, as would be appreciated by the skilled artisan. After dehydration, if any, the tissue sample can be either stored at 4°C for later use or immediately assembled into a flow cell for subsequent imagine steps.

### Example 1b

The following non-limiting example describes a sample preparation protocol for use in the methods of the present disclosure

First, a 5 µm FFPE microtome section of tissue is mounted onto a first microscope slide functionalized with 0.5% (3-Aminopropyl)trimethoxysilane. The first microscope slide was functionalized using methods standard in the art, as would be appreciated by the skilled artisan. As part of the sample preparation, the first microscope slide will serve as one surface of the flow cell that is to be assembled.

The slides are then baked at 60°C oven for 1 hour. Following baking, the paraffin on the slides is removed using xylene. Next, the previous paraformaldehyde fixation is undone by heating the slides at 100°C for 8 minutes in a target retrieval solution (e.g. 10mM Tris, 1 mM EDTA at pH 9.0). The length of heating can be adjusted for the different sample type (*e.g.* tissue, cell pellet, etc.). In a non-limiting example, the slides can be heated for 8 minutes in the case of cell pellets.

After heating, a solution of 0.0005% to 0.003% 200nm carboxylated microsphere fiducials stained in red, yellow, blue, and green are applied to the tissue and incubated for 5 minutes before washed off with 1X Phosphate Buffered Solution (PBS).

Without wishing to be bound by theory, the mixture of fiducials and FNDs are used in subsequent imaging steps for autofocusing and image registration.

Following addition of fiducials, the tissue sample is then post-fixed in 10% Neutral Buffered Formalin (NBF) for 5 minutes. Without wishing to be bound by theory, this post-fixing step is used to preserve the morphology of the tissue sample. The NBF is then neutralized with Tris Glycine buffer for 10 minutes and washed with 1X PBS.

In-situ hybridization (ISH) probe of the present disclosure are then denatured at 95°C for 2 minutes and crash cooled before being applied to the tissue sample at a 0.5 nM per probe concentration in a hybridization mix that contains Buffer R solution (Stock solution: 3.125% Dextran Sulfate, 0.25% BSA, 0.125mg/mL ssDNA, 2.5X SSC, 50% formamide; Working concentration: 2.5% Dextran Sulfate, 0.2% BSA, 0.1 mg/mL ssDNA, 2X SSC, 40% formamide) and RNAse inhibitor. The tissue sample is incubated with the ISH probes for 16-18 hours at 37° C.

Following incubation with the ISH probes, slides are then briefly dipped into 2x saline-sodium citrate (SSC) with 0.1% Tween-20 solution then incubated in 2 changes of 50% formamide and 2x SSC solution for at least 50 minutes to wash off excess, unbound ISH probes. Slides are then optionally dehydrated in an ethanol gradient using methods standard in the art, as would be appreciated by the skilled artisan. After dehydration, if any, the tissue sample can be either stored at 4°C for later use or immediately assembled into a flow cell for subsequent imaging steps.

### Example 2

The following non-limiting example describes a sample preparation protocol for use in the methods of the present disclosure

Day 0-Prior to the sample preparation protocol, microscope slides can be functionalized using the following protocol. First, the microscope slides are cleaned using a plasma machine using methods standard in the art, as would be appreciated by the skilled artisan. After cleaning, the slides are placed into a 0.5% (3-Aminopropyl)trimethoxysilane solution for soaking for 1 minute. After soaking, the slides are sonicated in the 0.5% (3-Aminopropyl)trimethoxysilane solution for 10 seconds. The soaking and sonication are then repeated twice, such that the total time the slides spend in the 0.5% (3-Aminopropyl)trimethoxysilane solution is about 3.5 minutes. The slides are then rinsed with water at least 3-4 times. Finally the slides are dried with under nitrogen.

Prior to the sample preparation, the biological sample (*e*.*g*. tissue sample) can be sectioned for use in the methods of the present disclosure. The biological sample is cut into a 5 µm FFPE microtome section and mounted onto a functionalized slide (see above). The slide with the mounted microtome section is then dried overnight at room temperature. If slides are not to be further processed immediately after drying, they are placed into storage at 4°C.

Day 1-The slide with the mounted microtome section (see above) is first baked at 60°C for 1 hour.

### Deparaffinizing

After baking, the slide is then immediately transferred into a solution of xylene and incubated for 5 minutes with agitation. The slide is then transferred to a fresh solution of xylene and incubated for another 5 minutes with agitation. The slide is then transferred to a 100% ethanol solution and incubated for 2 minutes. The slide is then transferred to a fresh solution of 100% ethanol and incubated for another 2 minutes. After this incubation, the slide is laid flat in a 60°C oven for 5 minutes to dry.

### Target Retrieval

1X target retrieval solution (prepared using diethyl pyrocarbonate (DEPC) treated water) is then preheated to 100°C. The solution can be preheated, for example, using a pressure cooker. The 1X target retrieval solution is not to be boiled for more than 15 minutes. Once the 1X target retrieval solution reaches 100°C, the slide is incubated in the 1x target retrieval solution for a time period that corresponds with the type of sample being processed. Incubation times for different sample types are shown in Table 1. After incubation at 100°C, the slide is immediately transferred to DEPC-treated water and incubated for 15 seconds with agitation. The slide is then transferred to a solution of 100% ethanol and incubated for three minutes. The slide is then removed from the ethanol solution and allowed for dry for 5 minutes.

### Tissue Permeabilization and Fiducial/FND application

After the target retrieval step, a hydrophobic barrier is drawn around the sample mounted on the slide, for example, using a PAP pen. Care is taken to ensure that the barrier is not too close to the tissue.

After the marking of the hydrophobic barrier, a 1 µg/mL proteinase K solution in PBS is prepared. The slide is then placed in a humidity tray that has been lined with paper wetted with DEPC water and heated at 40°C for at least 30 minutes. Once the slide is in the humidity tray, the proteinase K solution is applied to the biological sample mounted on the slide. The slide is then placed into an oven at 40°C and incubated according to the biological sample type. Incubation times for different sample types are shown in Table 2. After incubation, the proteinase K solution is removed from the biological sample. The slide is then washed with DEPC-treated water with agitation 3-6 times. Fresh DEPC-treated water is used at least for the last washing step.

Following washing of the slide, a Fiducial/FND mixture that has been vortexed for 30 seconds is applied to the biological sample on the slide. An exemplary Fiducial/FND mixture can be prepared by diluting 0.001% 200nm carboxylated microsphere fiducials in red, blue, and green to 0.001% and non-carboxylated fluorescent nano-diamonds (FND) to 0.00045% in 2x saline-sodium citrate (SSC) solution. This solution is then vortexed for 1 minute, then sonicated for 2 minutes, then vortexed again for 1 minute, then sonicated again for 2 minutes. The Fiducial/FND mixture is incubated with the biological sample for 5 minutes at room temperature. The slide is then washed with 1x PBS.

### Post Fix

Following tissue permeabilization and fiducial/FND application, the slide is incubated in a 10% Neutral Buffered Formalin (NBF) for 5 minutes. The slide is then incubated in Tris Glycine buffer for 5 minutes. The slide is then incubated in a fresh batch of Tris Glycine buffer for 5 minutes. Finally, the slide is incubated in 1x PBS for 5 minutes.

### Hybridization of ISH probes of the present disclosure

In-situ hybridization (ISH) probe of the present disclosure are then denatured at 95°C for 2 minutes and crash cooled on ice for 1 minute before being applied to the tissue sample at a 0.5 nM per probe concentration in a hybridization mix that contains Buffer R solution (Stock solution: 3.125% Dextran Sulfate, 0.25% BSA, 0.125mg/mL ssDNA, 2.5X SSC, 50% formamide; Working concentration: 2.5% Dextran Sulfate, 0.2% BSA, 0.1 mg/mL ssDNA, 2X SSC, 40% formamide) and RNAse inhibitor. The tissue sample is incubated with the ISH probes for 16-18 hours at 37° C in a container that was prewashed with RNase inhibitor and lined with paper wetted with 2x SSC solution.

### Day 2-Stringent washing of slide

After the hybridization of the ISH probes, the slide is removed from the oven and dipped briefly in 2x SSC solution. The slide is then incubated for 25 minutes with a 50% formamide in 2x SSC solution, wherein the formamide was preheated to 37°C. The slide is then incubated in a fresh aliquot of 50% formamide in 2x SSC solution for 25 minutes. The slide is then incubated in 2x SSC solution for 2 minutes. Finally, the slide is incubated in a fresh aliquot of 2x SSC solution for another two minutes.

### Optional Dehydration

After the stringent washing of the slide, the slide is optionally dehydrated in an ethanol gradient. First the slide is incubated in a 70% ethanol solution for 3 minutes, then is incubated in an 85% ethanol solution for 3 minutes, and then finally in a 100% ethanol solution for 3 minutes.

After dehydration, if any, the slide is either immediately assembled into a flow cell to be used in the imaging methods of the present disclosure or is stored at 4° C for later use.

### Example 3

The following non-limiting example describes a flow cell assembly protocol using the slides prepared in either Example 1 or Example 2 for use in the methods of the present disclosure

First, 300 µm thick coverglass is cleaned with isopropanol to remove dust, debris and/or water. 75 µm thick flow cell adhesive is then applied to the coverglass. The slide comprising the biological sample (prepared as described in Example 1 or Example 2) is then cleaned with isopropanol. The isopropanol is used to wipe around the biological sample mounted on the slide multiple times to remove any dust, and/or water. If the sample is not dehydrated, a kimwipe or suitable alternative is used to wipe around the mounted biological sample until the slide is free of liquid Care is given to ensure that the biological sample remained wet, including applying a compatible buffered solution if the biological sample appears to be drying out. The coverglass with adhesive is then pressed onto the slide with the mounted biological sample, for example, in a hydraulic press at a pressure of 250 psi for at least 30 seconds to form the flow cell. The coverglass is then further cleaned with isopropanol.

### Example 4

The following non-limiting examples described various solutions that can be used in the methods of the present disclosure

Sulfo-NHS Acetate Blocking Solution: 100 mM Sulfo-NHS acetate in 100 mM Sodium Phosphate pH 8.0.

Reporter Probe Solution: 5 nM reporter probes of the present disclosure, 8.75x SSPE Buffer, 0.5% Tween-20, 0.1% RNAse inhibitor in DEPC-treated water.

Low Salt Imaging Buffer: 1 M Tris-HCL pH 7.5, 5M Sodium Chloride and 0. 5% Tween-20 in DEPC-treated water.

Imaging Buffer: 98% Low Salt Imaging Buffer, 1% Protocatechuic Acid (PCA) and 1% Protocatechuate dioxygenase (PCD).

Membrane Stain Blocking Solution: 0.5% NaN₃ and 1% 4',6-diamidino-2-phenylindole (DAPI) in Buffer W.

Membrane Stain Solution: 5% NaN₃ and 1% DAPI in Buffer W further comprising at least one of a fluorescently labeled anti-CD298 antibody, a fluorescently labeled anti-CD3 antibody, a fluorescently labeled anti-CD20 antibody, and a fluorescently labeled anti-PanCK antibody.
Reporter Wash Buffer: 0.5% Tween-20 in 1x SSPE solution
Strip Wash Buffer: 0.0033x SSPE buffer and 0.5% Tween-20

### Example 5

The following is a non-limiting example of the analysis of biological samples using the sample preparation methods and imaging methods of the present disclosure

Biological samples, including samples comprising various cell lines such as CCRF-CEM cells, SUDHL4 cells, MDA-MB-468 cells, HS578T cells, EKVK cells, HCT116 cells, HOP92 cells, and COLO205 cells, as well as various FFPE samples, were prepared as described in the Examples above. Target analytes were then analyzed using the imaging methods described herein by sequential binding of reporter probes to ISH probes bound to target analytes in the biological samples.

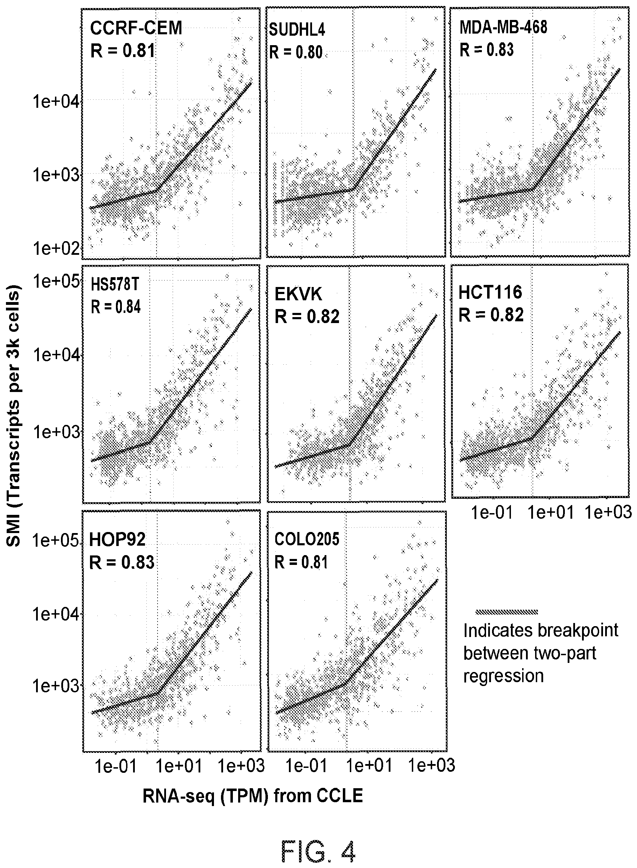

As a control, the abundance measurements made using the methods of the present disclosure were compared to publicly available abundance data collected using standard RNA-seq techniques. As shown in FIG. 4, the nucleic acid abundance data measured using the methods of the present disclosure showed high concordance with the standard RNA-seq data, for genes above limit of detection (defined as >1 FPKM expression level in Cancer Cell Encyclopedia database), demonstrating comparable sensitivity and specificity to that of standard RNA-seq techniques. Without wishing to be bound by theory, these results demonstrate that the methods of the present disclosure accurately measure target analyte abundance, with the added advantage that the spatial context of the target analytes is preserved and recorded, unlike with standard RNA-seq.

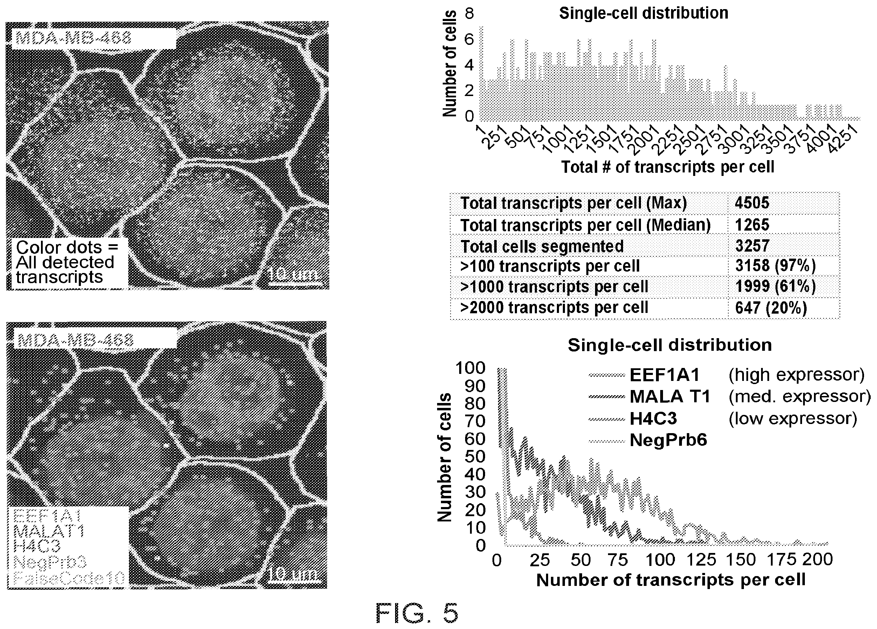

FIG. 5 shows images of individual target analytes detected in a biological sample comprising MDA-MB-468 cells, including the specific target analytes EEF1A1, MALAT1, H4C3. Also included is the signal recorded from a negative probe (NegPrb 6). The graphs and tables in FIG. 5 also demonstrate the number of cells that a particular number of transcripts detected using the methods of the present disclosure. As shown in FIG. 5, greater than 97% of the cells having at least 100 transcripts detected, with a median transcripts per cell of 1265. Moreover, FIG. 5 shows that the methods of the present disclosure were able to individually segment 3257 cells in the biological sample analyzed. Without wishing to be bound by theory, the results shown in FIG. 5 demonstrate that the methods of the present disclosure can determine the abundance and spatial location of individual target analytes in a biological sample with subcellular resolution, including target analytes that are highly abundant (e.g. EEF1A1 in FIG. 5), moderately abundant (MALAT1 in FIG. 5) and rare transcripts (e.g. H4C2 in FIG. 5).

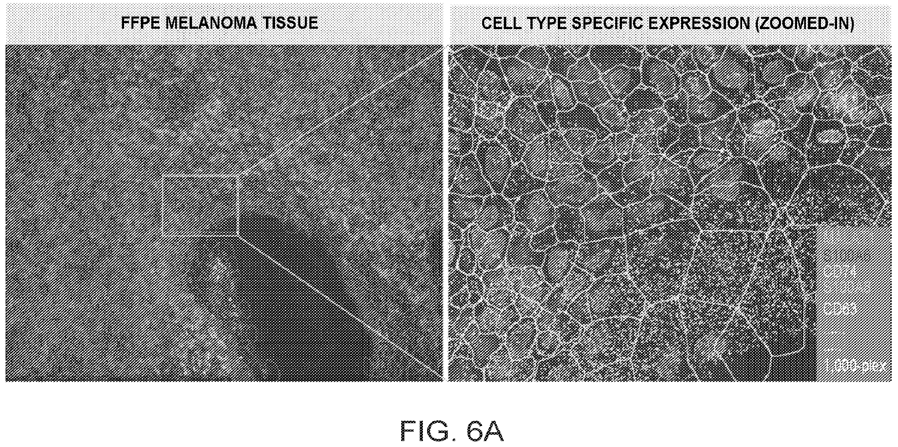

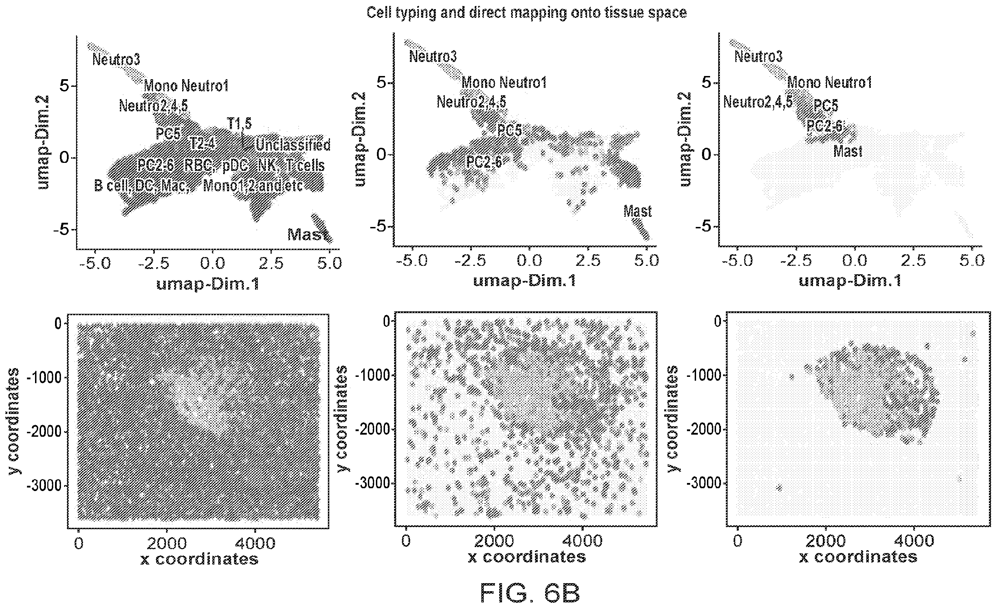

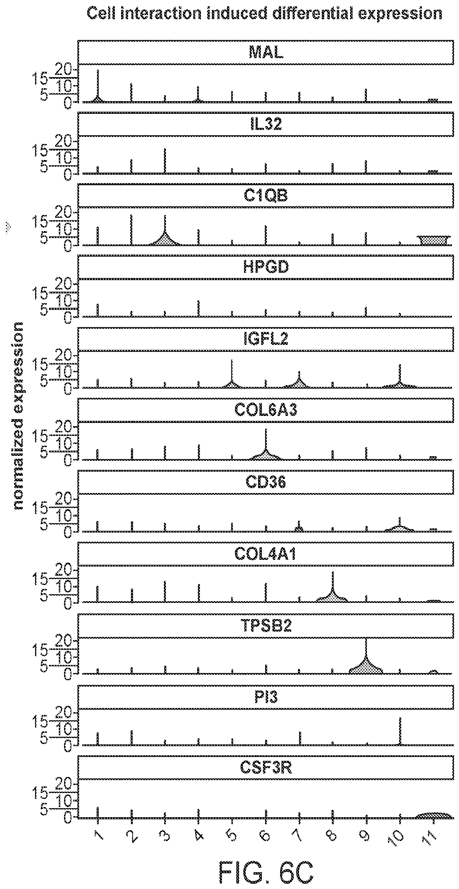

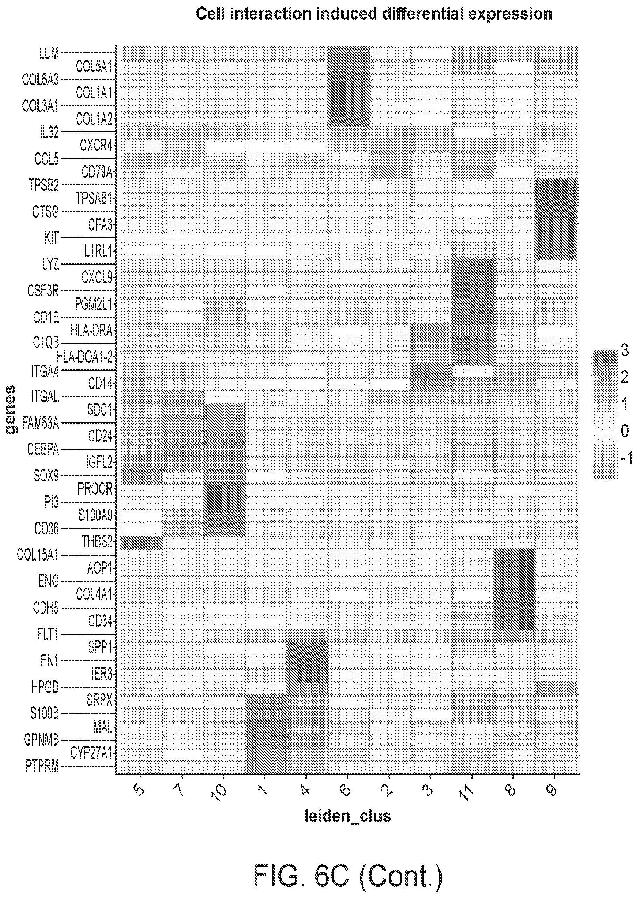

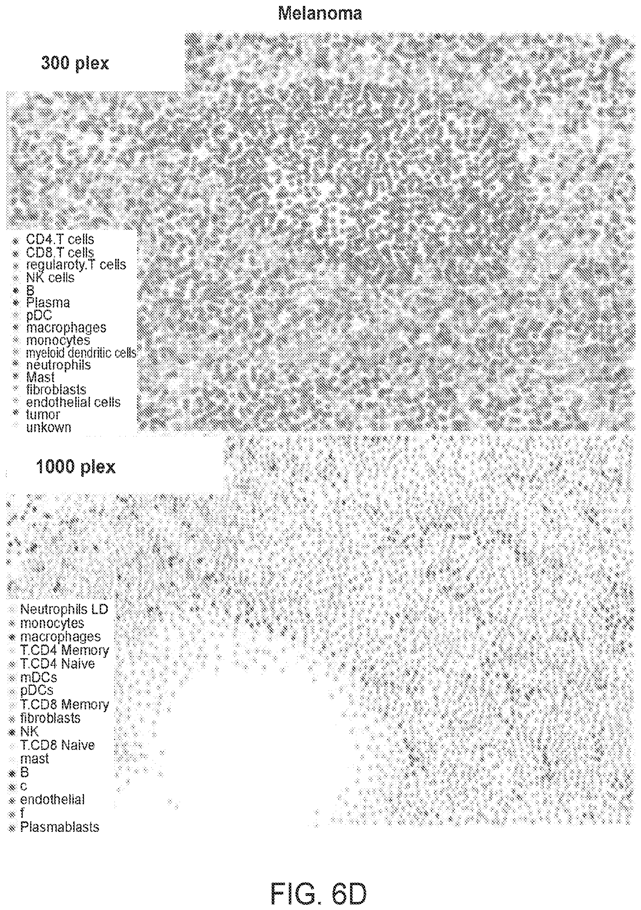

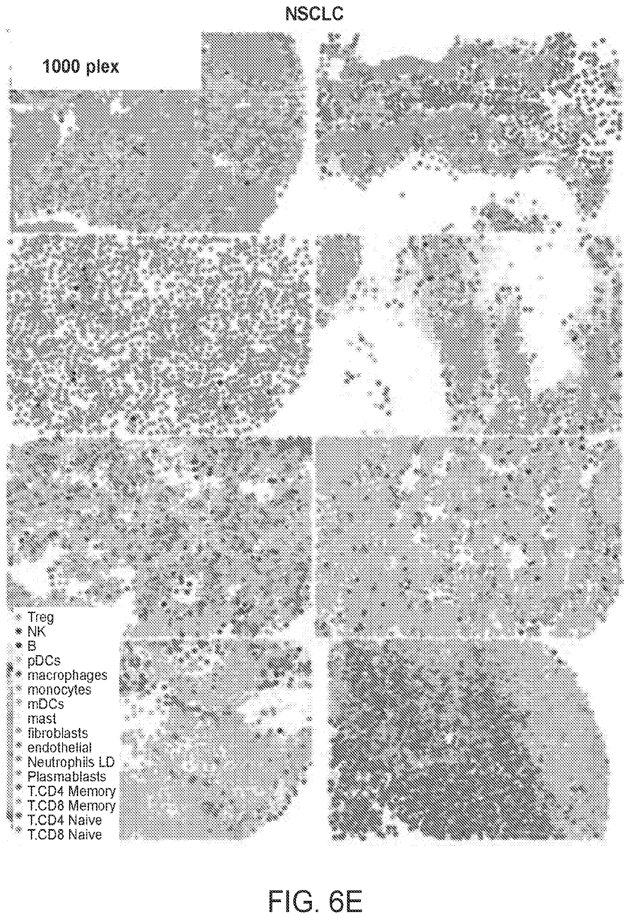

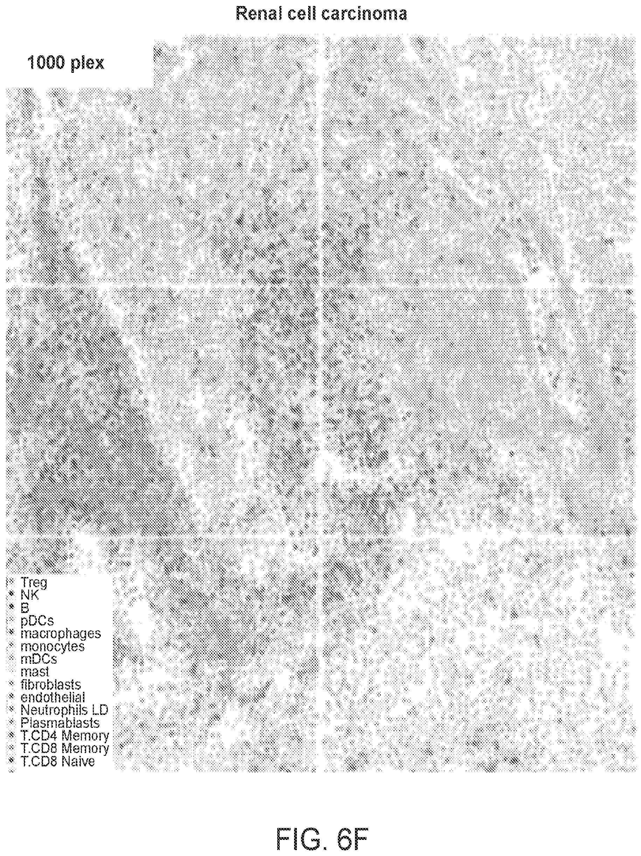

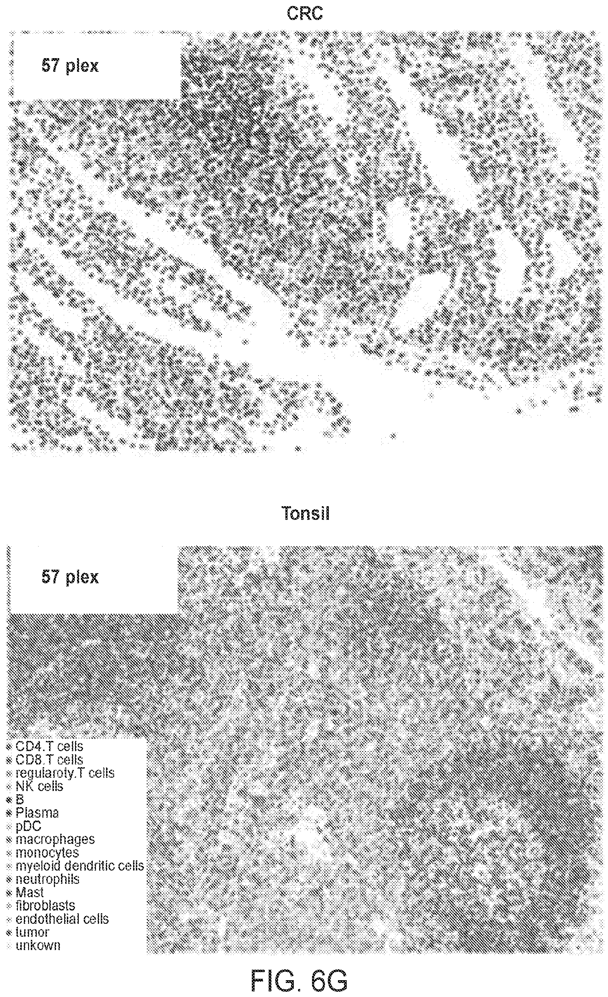

FIG. 6A shows images of an FFPE melanoma tissue sample analyzed according to the methods of the present disclosure. In this experiment, 1,000 different target analytes were measured and detected spatially with subcellular resolution. More specifically, 22 species of negative probes and 997 species of ISH probes targeting specific target nucleic acids were used Without wishing to be bound by theory, the ability of the methods of the present disclosure to determine the spatial abundance of 1,000 target analytes in a target tissue samples allows for a comprehensive spatial single cell analysis to be performed on a tissue sample, including cell typing and mapping, identification of cellular state, identification of cellular function, interaction analyses, differential expression analyses and hormone activity analyses, as is shown in FIGs. 6B and 6C. This analysis using the methods of the present disclosure was performed on additional FFPE samples, including non-small-cell lung cancer (NSCLC) FFPE samples, Renal cell Carcinoma FFPE samples, colorectal cancer (CRC) FFPE samples and Tonsil FFPE samples, whose cell typing results mapped to the tissue section is shown in FIGs. 6D-6G.

The results described in this example demonstrate that the methods of the present disclosure allow for the simultaneous quantification of spatial abundance for thousands of target analytes in biological samples, such as tissue samples, with subcellular resolution, thereby allowing the skilled artisan to perform a variety of different biological analyses at the single cell level.

### Equivalents

The foregoing description has been presented only for the purposes of illustration and is not intended to limit the disclosure to the precise form disclosed. The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs. All patents and publications cited in this specification are incorporated by reference.
The invention is further described by the following numbered paragraphs (paras):
1. A method of determining the abundance and spatial position of at least two target analytes in a biological sample,
   wherein the biological sample is prepared by:
   i) contacting the biological sample with at least one nucleic acid probe by incubating the mounted biological sample with a solution comprising a plurality of ISH probes,
      wherein the solution comprises at least two species of ISH probes,
      wherein at least one species of ISH probe comprises a unique target binding domain that binds to one of at least two target analytes and a unique barcode domain specific for the target analyte, wherein the barcode domain comprises at least one attachment position;
   ii) washing the biological sample,
   the method comprising:
   a) contacting the prepared biological sample with a plurality of reporter probes, wherein each reporter probe comprises at least one detectable label, thereby hybridizing a reporter probe to an attachment region of a barcode domain of at least one ISH probe hybridized to a target analyte in the biological sample;
   b) removing non-hybridized reporter probes from the biological sample,
   c) recording the identity and spatial position of the detectable labels of the hybridized reporter probes:
   d) removing the detectable labels of the hybridized reporter probes: and
   e) repeating steps (a)-(d) until each attachment position in the barcode domains of ISH probes hybridized to a target analyte in the biological have been hybridized to a reporter probe comprising at least one detectable label,
   thereby determining the abundance and spatial position of the at least two target analytes in the biological sample based on the sequence in which the detectable labels were recorded.
2. The method of para 1, wherein the at least two target analytes are target nucleic acid molecules, and
   wherein the target binding domain is a single-stranded polynucleotide comprising a nucleic acid sequence that is complementary to a target nucleic acid,
   wherein the target binding domain is about 35 to about 40 nucleotides in length, and
   wherein the target binding domain comprises D-DNA, and
   wherein the barcode domain is a single-stranded polynucleotide comprising at least one attachment region,
      wherein each attachment region comprises about one attachment sequence,
      wherein each of the attachment sequences is about 14 nucleotides in length,
      and wherein the sequences of each of the attachment sequences are different,
   and wherein the barcode domain comprises L-DNA.
3. The method of para 1, wherein the at least two target analytes are target protein molecules, and
   wherein the target binding domain comprises a protein, preferably wherein the protein is an antibody, or antigen binding fragment, that specifically binds to a target protein molecule.
4. The method of any one of the preceding paras, wherein the barcode domain comprises:
   i) at least two;
   ii) at least three;
   iii) at least four; or
   iv) at least five
   attachment regions.
5. The method of para 1, wherein the solution comprises at least one negative ISH probe that is designed not to specifically bind to any target analyte in the biological sample, preferably wherein the ISH probe comprises at least one Evaluation of the External RNA Controls Consortium (ERCC) sequence, or a complement thereof.
6. The method of para 5, wherein the negative ISH probe is used to determine the level of background noise in the biological sample.
7. The method of any one of the preceding paras, wherein the reporter probes comprise L-DNA.
8. The method of any one of the preceding paras, wherein the reporter probes comprise:
   a primary nucleic acid molecule comprising a first domain, a second domain and a photocleavable linker located between the first domain and the second domain,
   wherein the second domain of the primary nucleic acid molecule is hybridized to about six secondary nucleic acid molecules,
   wherein each secondary nucleic acid molecule comprises a first domain, a second domain and a photocleavable linker located between the first domain and the second domain,
   wherein the first domain of each of the secondary nucleic acid molecules is hybridized to the second domain of the primary nucleic acid molecule,
   wherein the second domain of each of the secondary nucleic acid molecules is hybridized to about five tertiary nucleic acid molecules,
   wherein each of the tertiary nucleic acid molecules comprise at least one detectable label, and
   wherein the primary nucleic acid molecule, the secondary nucleic acid molecules, and the tertiary nucleic acid molecules comprise L-DNA.
9. The method of any one of the preceding paras, wherein the at least one detectable label is a fluorescent moiety.
10. The method of any one of the preceding paras, the method further comprising prior to step (a):
   pretreating the biological sample by:
   i) incubating the biological sample in a Sulfo-NHS Acetate Blocking solution for about 15 minutes;
   ii) washing the biological sample with Reporter Wash Buffer;
   iii) incubating the biological sample in autofluorescence suppressor buffer and/or illuminating the biological sample with blue and/or UV light, thereby quenching sample autofluorescence via photobleaching; and
   iv) washing the biological sample with Reporter Wash Buffer.
11. The method of any one of the preceding paras, wherein step (a) comprises incubating the biological sample with a solution comprising the reporter probes at a concentration of 5 nM, 8.75x SSPE solution, 0.5% Tween-20 and, optionally 0.1% RNase inhibitor, in DEPC-treated water for at least about 15 minutes.
12. The method of any one of the preceding paras, wherein step (b) comprises washing the biological sample with Reporter Wash Buffer.
13. The method of any one of the preceding paras, wherein step (c) comprises:
   i) immersing the biological sample in Imaging Buffer; and
   ii) imaging the biological sample to record the identity and spatial position of the detectable labels of the hybridized reporter probes.
14. The method of any one of the preceding paras, wherein step (d) comprises:
   i) performing at least one of or both of:
      illuminating the biological sample with UV light sufficient to cleave photocleavable linker moieties in the hybridized reporter probes; and
      washing the biological sample with Strip Wash Buffer,
   optionally, step (d) further comprises:
   iii) immersing the biological sample in Imaging Buffer; and
   iv) imaging the sample to ensure that there are no remaining detectable labels.
15. The method of any one of the preceding paras, further comprising performing morphology scanning of the biological sample to determine one or more regions of interest, preferably wherein performing morphology scanning comprises at least one of:
   i) staining the biological sample with a membrane specific-fluorescent staining solution and imaging the biological sample to identify the spatial location of cellular membranes within the sample;
   ii) staining the biological sample with a nuclear-specific fluorescent staining solution and imaging the biological sample to identify the spatial location of cellular nuclei in the sample; and
   iii) performing cell segmentation.
16. The method of any one of the preceding paras, wherein the biological sample is further prepared prior to contacting the biological sample with at least one nucleic acid probe by:
   aa) mounting a biological sample onto a functionalized microscope slide thereby producing a mounted biological sample, wherein the biological sample is a formalin fixed paraffin embedded (FFPE) microtome section;
   bb) baking the mounted biological sample;
   cc) deparaffinizing the mounted biological sample;
   dd) performing a target retrieval reaction on the mounted biological sample;
   ee) permeabilizing the mounted biological sample;
   ff) applying at least one fiducial marker to the mounted biological sample; and
   gg) fixing the mounted biological sample
17. The method of any one of the preceding paras, further comprising after step (ii), assembling the mounted biological sample into a flow cell.
18. The method of any one of the preceding paras, wherein the functionalized microscope slide is a positively charged microscope, preferably wherein the functionalized microscope slide is a (3-Aminopropyl)trimethoxysilane (APTMS)-functionalized microscope slide.
19. The method of any one of the preceding paras, wherein the biological sample is an FFPE microtome section of a human tissue sample.
20. The method of any one of paras 16-19, wherein step (bb) comprises baking the mounted biological sample at about 60°C for about 1 hour.
21. The method of any one of paras 16-20, wherein step (cc) comprises:
   i) incubating the mounted biological sample in a first solution of xylene for about 5 minutes:
   ii) incubating the mounted biological sample in a second solution of xylene for about 5 minutes;
   iii) incubating the mounted biological sample in a first 100% ethanol solution for about 2 minutes;
   iv) incubating the mounted biological sample in the second 100% ethanol solution for about 2 minutes; and
   v) drying the mounted biological sample at about 60°C for about 5 minutes.
22. The method of any one of paras 16-21, wherein step (dd) comprises:
   i) incubating the mounted biological sample in target retrieval solution at about 100°C;
   ii) incubating the mounted biological sample in DEPC-treated water for about 15 seconds;
   iii) incubating the mounted biological sample in a solution of 100% ethanol for about 3 minutes; and
   iv) drying the mounted biological sample.
23. The method of para 22, wherein the mounted biological sample is incubated in the target retrieval solution for a time period as put forth in Table 1.
24. The method of para 22 or para 23, wherein the target retrieval solution comprises TRIS and EDTA solution and has a pH of about 9.
25. The method of any one of paras 16-24, wherein step (ee) comprises:
   i) incubating the mounted biological sample at about 40°C in a proteinase solution, wherein the proteinase solution comprises protease K;
   ii) washing the biological sample with a first aliquot of DEPC-treated water; and
   iii) washing the biological sample with a second aliquot of DEPC-treated water.
26. The method of para 25, wherein the mounted biological sample is incubated in the proteinase K solution for a time period as put forth in Table 2.
27. The method of any one of paras 16-26, wherein step (ff) comprises:
   i) incubating the mounted biological sample in a solution comprising at least one fiducial marker for about 5 minutes at about room temperature, wherein the solution comprising at least one fiducial marker is a solution comprising carboxylated microspheres stained in red, yellow, blue and/or green at a concentration of about 0.0005% to about 0.003% in 2x SSCT solution; and
   ii) washing the mounted biological with 1x PBS.
28. The method of any one of paras 16-27, wherein step (gg) comprises
   i) incubating the mounted biological sample in a 10% NBF for about 1 minutes;
   ii) incubating the mounted biological sample in a first tris glycine buffered solution for about 5 minutes;
   iii) incubating the mounted biological sample in a second tris glycine buffered solution for about 5 minutes; and
   iv) incubating the mounted biological sample in 1x PBS for about 5 minutes.
29. The method of any one of paras 16-28, further comprising after step (gg), incubating the mounted biological sample in a blocking solution, wherein incubating the mounted biological sample in a blocking solution comprises:
   i) incubating the mounted biological sample in a Sulfo-NHS-acetate/Tween20 solution for about 15 minutes, wherein the Sulfo-NHS-acetate/Tween20 solution comprises about 100 mM Sulfo-NHS-acetate, about 0.5% Tween20 in about 100 mM sodium phosphate pH 8; and
   ii) incubating the mounted biological sample in 1x PBS for about 5 minutes.
30. The method of any of the preceding paras, wherein incubating the mounted biological sample with a solution comprising a plurality of ISH probes comprises:
   incubating the mounted biological sample with a solution comprising a plurality of ISH probes for about 16 to about 18 hours at about 37°C, thereby hybridizing at least one ISH probe to a target analyte in the biological sample.
31. The method of any of the preceding paras, wherein washing the biological sample comprises:
   i) incubating the mounted biological sample with a first 2x SSC solution;
   ii) incubating the mounted biological sample in a first formamide solution;
   iii) incubating the mounted biological sample with a second formamide solution;
   iv) incubating the mounted biological sample with a second 2x SSC solution; and
   v) incubating the mounted biological sample with a third 2x SSC solution.

## Claims

1. A method of performing morphology scanning on a biological sample comprising determining the abundance and/or spatial position of at least two target analytes, the method comprising:
i) contacting the biological sample with at least one in situ hybridization (ISH) probe, wherein the at least one ISH probe comprises a unique target binding domain that binds to one of the at least two target analytes in the biological sample and a unique barcode domain specific for the one target analyte, thereby hybridizing the at least one ISH probe to the one target analyte in the biological sample, wherein the unique target binding domain comprises an antibody, and wherein the barcode domain comprises at least one attachment position;
ii) contacting the biological sample with a plurality of reporter probes, wherein each reporter probe comprises at least one detectable label, thereby hybridizing at least one reporter probe to an attachment region of the barcode domain of at least one ISH probe hybridized to the one target analyte in the biological sample;
iii) removing non-hybridized reporter probes from the biological sample;
iv) recording an identity and/or spatial position of the at least one detectable label of the at least one hybridized reporter probe;
v) removing the at least one detectable label of the hybridized reporter probes; and
vi) repeating steps (ii)-(v) until each attachment position in the barcode domains of ISH probes hybridized to a target analyte in the biological sample have been hybridized to a reporter probe comprising at least one detectable label,
thereby determining the abundance and/or spatial position of the at least two target analytes in the biological sample based on the sequence in which the detectable labels were recorded, thereby determining one or more regions of interest.

2. The method of claim 1, wherein the at least two target analytes are target protein molecules.

3. The method of claim 1, wherein the solution comprises at least one negative ISH probe that is designed not to specifically bind to any target analyte in the biological sample.

4. The method of claim 3, wherein the negative ISH probe is used to determine a level of background noise in the biological sample.

5. The method of any one of the preceding claims, wherein performing morphology scanning further comprises at least one of:
i) Staining the biological sample with a membrane specific-fluorescent staining solution and imaging the biological sample to identity the spatial location of cellular membranes within the sample;
ii) Staining the biological sample with a nuclear-specific fluorescent staining solution and imaging the biological sample to identify the spatial location of cellular nuclei in the sample; and
iii) performing cell segmentation.
